# EUROPEAN PATENT APPLICATION

(11) **EP 4 446 420 A1**
(43) Date of publication of application: **16.10.2024**
(21) Application number: 22904228.8
(22) Date of filing: 06.12.2022
(51) Int. Cl.: C12P 13/04, C12P 19/04, C12N 5/07, C12N 5/077, C12N 5/10, C12N 1/00, C12N 1/12, C12N 1/13, C12N 1/15, C12N 1/19, C12N 1/21, C12N 15/31, C12N 15/52, C12N 15/53, C12Q 1/04, C12Q 1/68, C12P 7/56, C12P 7/62, A23L 13/00

(54) **LIVING ORGANISM HAVING L-LACTIC ACID UTILIZING CHARACTERISTICS, AND RESOURCE RECYCLING METHOD USING SAME**

(30) Priority: 07.12.2021 JP 2021198872
(71) Applicant: Tokyo Women's Medical University, Tokyo 162-8666 (JP)
(72) Inventor: HASUNUMA, Tomohisa, Kobe-shi, Hyogo 657-8501 (JP); KATO, Yuichi, Kobe-shi, Hyogo 657-8501 (JP); INABE, Kosuke, Kobe-shi, Hyogo 657-8501 (JP); KONDO, Akihiko, Kobe-shi, Hyogo 657-8501 (JP); SHIMIZU, Tatsuya, Tokyo 162-8666 (JP); HARAGUCHI, Yuji, Tokyo 162-8666 (JP)
(74) Representative: Vossius & Partner Patentanwälte Rechtsanwälte mbB
(86) International application number: PCT/JP2022/044962
(87) International publication number: WO 2023/106300

(57) **Abstract**

According to the present disclosure, there is provided a system for culturing animal cells using a component derived from an organism such as algae as a nutrient source, and reusing the culture waste liquid. The present disclosure provides an organism or a cultured cell which undergoes a modification, in which the modification includes imparting or enhancing L-lactate utilization ability in the organism or the cultured cell. In addition, the present disclosure provides a method for culturing at least two types of cells including a cell X and a cell Y in a circulation manner, the method including: a step (a) of providing a component excreted from the cell X to the cell Y; a step (b) of providing a component derived from the cell Y to the cell X; a step (c) of culturing the cell X and the cell Y; and a step (d) of repeating the steps (a) to (c) as necessary, in which at least one of the components excreted from the cell X is an assimilable component of the cell Y, and at least one of the components derived from the cell Y is a nutritional component of the cell X.

## Description

### Technical Field

The present disclosure relates to a resource circulation method using cells. More specifically, the present disclosure relates to an organism having L-lactate usability. More specifically, the present disclosure relates to a resource circulation method using an organism having L-lactate usability, and a technique for culturing animal cells using the same to produce cultured meat or obtain a desired product.

### Background Art

In the current food production in which some of individuals of plants and animals such as grains or livestock are used as food, a lot of waste occurs. In recent years, cultured meat produced by culturing cells of edible parts has attracted worldwide attention and has been actively developed. On the other hand, it is problematic that component of a medium used for cell culture are originally derived from grains or livestock, and are significantly expensive. Furthermore, it is also required to take measures against a large amount of culture waste liquid caused by the spread of cultured meat in the future.

### Summary of Invention

### Solution to Problem

Therefore, by utilizing ability of cells, the present disclosure provides a method for establishing a production technique of only an edible part by circular cell culture using photosynthesis as a driving force and by three-dimensional organization of cells.

Accordingly, the present disclosure provides the following.

### (Item 1)

An organism or a cultured cell which undergoes a modification, in which the modification includes imparting or enhancing L-lactate utilization ability in the organism or the cultured cell.

### (Item 2)

The organism or the cultured cell according to the above item, in which the organism or the cultured cell includes blue-green algae, microalgae, a yeast, E. coli, Bacillus subtilis, corynebacterium, actinomycetes, filamentous fungus, Bacillus subtilis, and Lactobacillus.

### (Item 3)

The organism or the cultured cell according to any one of the above items, in which the modification includes introduction of a gene that performs L-lactate utilization.

### (Item 4A)

The organism or the cultured cell according to any one of the above items, in which the modification includes introduction of a gene that encodes NAD-independent L-lactate dehydrogenase (EC 1.1.2.3).

### (Item 4)

The organism or the cultured cell according to any one of the above items, in which the modification includes introduction of lldD gene derived from E. coli.

### (Item 5)

The organism or the cultured cell according to any one of the above items, in which the modification includes introduction of a gene that imparts or enhances membrane permeability of L-lactate.

### (Item 6A)

The organism or the cultured cell according to any one of the above items, in which the modification includes introduction of a gene that encodes lactate permease.

### (Item 6)

The organism or the cultured cell according to any one of the above items, in which the modification includes introduction of lldP gene derived from E. coli.

### (Item 7)

The organism or the cultured cell according to any one of the above items, in which the modification includes attenuation or deletion of a gene that performs L-lactate or D-lactate synthesis.

### (Item 8A)

The organism or the cultured cell according to any one of the above items, in which the modification includes attenuation or deletion of a gene that encodes D-lactate dehydrogenase.

### (Item 8)

The organism or the cultured cell according to any one of the above items, in which the modification includes attenuation or deletion of ldhA gene.

### (Item 9)

The organism or the cultured cell according to any one of the above items, in which the organism or the cultured cell has a pyruvic acid metabolism system and/or a TCA cycle.

### (Item 9A)

The organism or the cultured cell according to any one of the above items, in which the organism or the cultured cell has a synthetic pathway of succinic acid, 2-oxoglutaric acid, 3-hydroxypropionic acid, acrylic acid, and/or acetic acid.

### (Item 10)

The organism or the cultured cell according to any one of the above items, in which the organism or the cultured cell has an amino acid synthetase and/or an amino acid synthetic system, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane.

### (Item 11)

The organism or the cultured cell according to any one of the above items, in which the amino acid includes glutamine, glutamic acid, alanine, valine, and leucine.

### (Item X1)

A method for culturing at least two types of cells including a cell X and a cell Y in a circulation manner, the method including:
a step (a) of providing a component excreted from the cell X to the cell Y;
a step (b) of providing a component derived from the cell Y to the cell X;
a step (c) of culturing the cell X and the cell Y; and
a step (d) of repeating the steps (a) to (c) as necessary, in which
at least one of the components excreted from the cell X is a usable component of the cell Y, and
at least one of the components derived from the cell Y is a nutritional component of the cell X.

### (Item X1A)

The method according to any one of the above items, in which the copmonent excreted from the cell X include L-lactate.

### (Item X2)

A method for producing a desired product, the method including a step of performing the method descried in the above item,
in which at least one of the cell X or Y produces the desired product.

### (Item X3)

The method according to any one of the above items, in which the cells X and Y include animal cells and blue-green algae cells.

### (Item X4)

The method according to any one of the above items, in which the component excreted from the cell X and the component derived from the cell Y include L-lactate and glutamine or glutamate. respectively.

### (Item X5)

The method according to any one of the above items, in which the culturing step includes culturing the cell X and the cell Y in different places.

### (Item A1)

A method for culturing animal cells by recycling a component excreted from an animal cell culture by an organism, the method including:
a step (a) of consuming the excreted component, the organism having utilization ability of the component;
a step (b) of harvesting a nutrient source of the animal cells from a culture solution of the organism;
a step (c) of culturing the animal cells using the nutrient source; and
a step (d) of repeating the steps (a) to (c) as necessary.

### (Item A2)

The method according to the above item, in which the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B 1, vitamin B2, vitamin B3, vitamin B5, vitamin B6, folate, choline, starch, glycogen, and cellulose.

### (Item A3)

The method according to any one of the above items, in which the organism has an ability to utilize the excreted component to produce the nutrient source, or the organism is modified to impart or enhance utilization ability of the component.

### (Item A4)

The method according to any one of the above items, in which the excreted component includes L-lactate, carbon dioxide, ammonium, urea, or phosphate.

### (Item A5)

The method according to any one of the above items, in which the excreted component is L-lactate, ammonium, or urea, and is toxic to the animal cells.

### (Item A6)

The method according to any one of the above items, in which the organism has a pyruvic acid metabolism system and/or a TCA cycle.

### (Item A7)

The method according to any one of the above items, in which the organism has a synthetic pathway of succinic acid, 2-oxoglutaric acid, 3-hydroxypropionic acid, acrylic acid, and/or acetic acid.

### (Item B 1)

A method for producing cultured meat by recycling the excreted component from an animal cell culture by an organism and culturing animal cells, the method including:
a step (a) of consuming the excreted component, the organism having utilization ability of the component;
a step (b) of harvesting a nutrient source of the animal cells from the organism;
a step (c) of culturing the animal cells using the nutrient source;
a step (d) of repeating the steps (a) to (c) as necessary; and
a step (e) of subjecting the cultured animal cells to three-dimensional organization for molding the three-dimensionally organized animal cells into cultured meat.

### (Item B2)

The method according to the above item, in which the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B 1, vitamin B2, vitamin B3, vitamin B5, vitamin B6, folate, choline, starch, glycogen, and cellulose.

### (Item B3)

The method according to any one of the above items, in which the organism has an ability to utilize the excreted component to produce the nutrient source, or the organism is modified to impart or enhance utilization ability of the component.

### (Item B4)

The method according to any one of the above items, in which the excreted component includes L-lactate, carbon dioxide, ammonium, urea, or phosphate.

### (Item B5)

The method according to any one of the above items, in which the excreted component is L-lactate, ammonium, or urea, and is toxic to the animal cells.

### (Item B6)

The method according to any one of the above items, in which the organism has a pyruvic acid metabolism system and/or a TCA cycle.

### (Item B7)

The method according to any one of the above items, in which the organism has a synthetic pathway of succinic acid, 2-oxoglutaric acid, 3-hydroxypropionic acid, acrylic acid, and/or acetic acid.

### (Item C1)

A method for producing an amino acid and/or a desired product in an organism or a cultured cell using L-lactate excreted from an animal cell culture, the method including:
a step of providing the L-lactate to the organism or the cultured cell, the organism or the cultured cell having L-lactate utilization ability or being modified to impart or enhance L-lactate utilization ability;
a step of culturing the organism or the cultured cell under conditions in which L-lactate is utilized; and
a step of harvesting the amino acid as necessary.

### (Item C2)

The method according to the above item, in which the organism or the cultured cell includes blue-green algae, microalgae, a yeast, E. coli, Bacillus subtilis, corynebacterium, actinomycetes, filamentous fungus, Bacillus subtilis, and Lactobacillus.

### (Item C3)

The method according to any one of the above items, in which the modification includes introduction of a gene that utilizes L-lactate.

### (Item C4A)

The method according to any one of the above items, in which the modification includes introduction of a gene that encodes NAD-independent L-lactate dehydrogenase (EC 1.1.2.3).

### (Item C4)

The method according to any one of the above items, in which the modification includes introduction of lldD gene derived from E. coli.

### (Item C5)

The method according to any one of the above items, in which the modification includes introduction of a gene that imparts or enhances membrane permeability of L-lactate.

### (Item C6A)

The method according to any one of the above items, in which the modification includes introduction of a gene that encodes lactate permease.

### (Item C6)

The method according to any one of the above items, in which the modification includes introduction of lldP gene derived from E. coli.

### (Item C7)

The method according to any one of the above items, in which the modification includes attenuation or deletion of a gene that synthesizes L-lactate or D-lactate.

### (Item C8A)

The method according to any one of the above items, in which the modification includes attenuation or deletion of a gene that encodes D-lactate dehydrogenase.

### (Item C8)

The method according to any one of the above items, in which the modification includes attenuation or deletion of ldhA gene.

### (Item C9)

The method according to any one of the above items, in which the organism or the cultured cell has a pyruvic acid metabolism system and/or a TCA cycle.

### (Item C9A)

The method according to any one of the above items, in which the organism or the cultured cell has a synthetic pathway of succinic acid, 2-oxoglutaric acid, 3-hydroxypropionic acid, acrylic acid, and/or acetic acid.

### (Item C10)

The method according to any one of the above items, in which the organism or the cultured cell has an amino acid synthetase and/or an amino acid synthetic system, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane.

### (Item C10A)

The method according to any one of the above items, in which the amino acid includes glutamine, glutamic acid, alanine, valine, and leucine.

### (Item C11)

The method according to any one of the above items, in which the desired product includes D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, and butane.

### (Item D1)

A method for producing a processed product of an amino acid and/or a desired product produced by an organism or a cultured cell using L-lactate excreted from an animal cell culture, the method including:
a step of providing the L-lactate to the organism or the cultured cell, the organism or the cultured cell having L-lactate utilization ability or being modified to impart or enhance L-lactate utilization ability in the organism or the cultured cell;
a step of culturing the organism or the cultured cell under conditions in which L-lactate is utilized;
a step of recovering the amino acid and/or the desired product; and
a step of processing the amino acid and/or the desired product.

### (Item D2)

The method according to the above item, in which the organism or the cultured cell includes blue-green algae, microalgae, a yeast, E. coli, Bacillus subtilis, corynebacterium, actinomycetes, filamentous fungus, Bacillus subtilis, and Lactobacillus.

### (Item D3)

The method according to any one of the above items, in which the modification includes introduction of a gene that utilizes L-lactate.

### (Item D4A)

The method according to any one of the above items, in which the modification includes introduction of a gene that encodes NAD-independent L-lactate dehydrogenase (EC 1.1.2.3).

### (Item D4)

The method according to any one of the above items, in which the modification includes introduction of lldD gene derived from E. coli.

### (Item D5)

The method according to any one of the above items, in which the modification includes introduction of a gene that imparts or enhances membrane permeability of L-lactate.

### (Item D6A)

The method according to any one of the above items, in which the modification includes introduction of a gene that encodes lactate permease.

### (Item D6)

The method according to any one of the above items, in which the modification includes introduction of lldP gene derived from E. coli.

### (Item D7)

The method according to any one of the above items, in which the modification includes attenuation or deletion of a gene that synthesizes L-lactate or D-lactate.

### (Item D8A)

The method according to any one of the above items, in which the modification includes attenuation or deletion of a gene that encodes D-lactate dehydrogenase.

### (Item D8)

The method according to any one of the above items, in which the modification includes attenuation or deletion of ldhA gene.

### (Item D9)

The method according to any one of the above items, in which the organism or the cultured cell has a pyruvic acid metabolism system and/or a TCA cycle.

### (Item D9A)

The method according to any one of the above items, in which the organism or the cultured cell has a synthetic pathway of succinic acid, 2-oxoglutaric acid, 3-hydroxypropionic acid, acrylic acid, and/or acetic acid.

### (Item D10)

The method according to any one of the above items, in which the organism or the cultured cell has an amino acid synthetase and/or an amino acid synthetic system, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane.

### (Item D10A)

The method according to any one of the above items, in which the amino acid includes glutamine, glutamic acid, alanine, valine, and leucine.

### (Item D11)

The method according to any one of the above items, in which the desired product includes D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, and butane.

### (Item E1)

A method for breeding an organism or a cultured cell using a component excreted from an animal cell culture, the method including:
a step (A) of providing a component excreted from animal cells to a candidate organism or cultured cell;
a step (B) of determining whether the organism or the cultured cell consumes the excreted a component or enhances the consumption; and
a step (C) of selecting an organism or a cultured cell in which consumption is imparted or enhanced among the organisms or cultured cells, in which
the selected organism or cultured cell is determined to have utilization ability of a component or to be modified to impart or enhance utilization ability of a component in the organism or the cultured cell.

### (Item E2)

The method according to any one of the above items, further including producing a nutrient source through utilization.

### (Item E3)

The method according to any one of the above items, in which the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B 1, vitamin B2, vitamin B3, vitamin B5, vitamin B6, folate, choline, starch, glycogen, and cellulose.

### (Item E4)

The method according to any one of the above items, in which a component to be excreted includes L-lactate, carbon dioxide, ammonium, urea, or phosphate.

### (Item E5)

The method according to any one of the above items, in which a component to be excreted is L-lactate, ammonium, or urea, and is toxic to the animal cells.

### (Item E6)

The method according to any one of the above items, in which the organism has a pyruvic acid metabolism system and/or a TCA cycle.

### (Item E6A)

The method according to any one of the above items, in which the organism has a synthetic pathway of succinic acid, 2-oxoglutaric acid, 3-hydroxypropionic acid, acrylic acid, and/or acetic acid.

### (Item F 1)

A method for maintaining an organism or a cultured cell using a component excreted from an animal cell culture, the method including:
a step (a) of causing the organism or the cultured cell to consume a component to be excreted, the organism or the cultured cell having utilization ability of a component or being modified to impart or enhance utilization ability of a component in the organism or the cultured cell;
a step (b) of recovering a nutrient source of the animal cells from the organism or the cultured cell as necessary;
a step (c) of culturing the animal cells using the nutrient source as necessary; and
a step (d) of providing a component excreted from the animal cells to the organism or the cultured cell.

### (Item F2)

The method according to any one of the above items, in which the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B 1, vitamin B2, vitamin B3, vitamin B5, vitamin B6, folate, choline, starch, glycogen, and cellulose.

### (Item F3)

The method according to any one of the above items, in which a component to be excreted includes L-lactate, carbon dioxide, ammonium, urea, or phosphate.

### (Item F4)

The method according to any one of the above items, in which a component to be excreted is L-lactate, ammonium, or urea, and is toxic to the animal cells.

### (Item F5)

The method according to any one of the above items, in which the organism has a pyruvic acid metabolism system and/or a TCA cycle.

### (Item F5A)

The method according to any one of the above items, in which the organism has a synthetic pathway of succinic acid, 2-oxoglutaric acid, 3-hydroxypropionic acid, acrylic acid, and/or acetic acid.

### (Item G1)

A gene used for breeding the organism or the cultured cell which undergoes a modification according to any one of the above items or a kit containing the gene.

### (Item H1)

A method for evaluating a modified strain, the method including:
a step of preparing the organism or the cultured cell according to any one of the above items; and
a step of confirming the presence of the modification in the organism or the cultured cell.

### (Item H2)

The method according to any one of the above items, in which the step of confirming the presence of the modification includes supplying carbon dioxide used in photosynthesis to the organism or the cultured cell at a constant concentration.

### (Item I1)

A method for producing a plastic raw material from an organism or a cultured cell using L-lactate excreted from an animal cell culture, the method including:
a step of providing the L-lactate to the organism or the cultured cell, the organism or the cultured cell having L-lactate utilization ability or being modified to impart or enhance L-lactate utilization ability in the organism or the cultured cell;
a step of culturing the organism or the cultured cell under conditions in which L-lactate is utilized; and
a step of recovering a component used as a plastic raw material from the organism or the cultured cell, in which
the organism or the cultured cell is capable of synthesizing a component used as a plastic raw material, or a gene required for synthesis of a component used as a plastic raw material is introduced into the organism or the cultured cell.

### (Item 11)

A method for producing a lactate derivative or a derivative thereof, the method including:
a step of providing L-lactate to an organism which undergoes a modification, the modification including imparting or enhancing L-lactate utilization ability in the organism;
a step of culturing the organism under conditions in which L-lactate is utilized; and
a step of recovering a desired lactate derivative or a derivative thereof as necessary.

### (Item J2)

A method for producing pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid, the method including:
a step of providing L-lactate to an organism which undergoes a modification, the modification including imparting or enhancing L-lactate utilization ability in the organism;
a step of culturing the organism under conditions in which L-lactate is utilized; and
a step of recovering a desired pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid as necessary.

### (Item J3)

The method according to the above item, further including a step of recovering a substance released extracellularly.

### (Item J4)

A method for producing a processed product of pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid, the method including:
a step of providing L-lactate to an organism which undergoes a modification, the modification including imparting or enhancing L-lactate utilization ability in the organism;
a step of culturing the organism under conditions in which L-lactate is utilized;
a step of recovering a desired pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid as necessary; and
a step of processing the pyruvic acid, acetyl CoA, phosphoenolpyruvate, succinic acid, 2-oxoglutaric acid, hydroxypropionic acid, acrylic acid, or acetic acid, or the derivative of pyruvic acid, acetyl CoA, phosphoenolpyruvate, succinic acid, 2-oxoglutaric acid, hydroxypropionic acid, acrylic acid, or acetic acid.

In the present disclosure, it is intended that one or a plurality of features can be provided in further combination in addition to the specified combination. Note that still further embodiments and advantages of the present disclosure will be appreciated by those skilled in the art upon reading and understanding the following detailed description, if necessary.

Note that features and remarkable operations and effects of the present disclosure other than those described above will be apparent to those skilled in the art with reference to the following embodiments and drawings of the invention.

### Advantageous Effects of Invention

According to the present disclosure, a modified organism having L-lactate usability can be provided. In addition, according to the method of the present disclosure, it is possible to provide a system for culturing animal cells using a component derived from algae as a nutrient source and for reusing the culture waste liquid, and it is possible to construct a meat production system having a significantly smaller environmental burden than a conventional food production system by breeding livestock using grains as feed.

### Brief Description of Drawings

FIG. 1 is a schematic view showing a model for introducing a lactate operon gene into blue-green algae in an embodiment of the present disclosure.
FIG. 2 is an amino acid sequence of LldD in Escherichia coli (SEQ ID NO: 1).
FIG. 3 is an amino acid sequence ofLldP in Escherichia coli (SEQ ID NO: 2).
FIG. 4 is a nucleic acid sequence of codon-optimized lldD using Synechococcus elongatus as a host (SEQ ID NO: 3).
FIG. 5 is a nucleic acid sequence of codon-optimized lldP using Synechococcus elongatus as a host (SEQ ID NO: 4).
FIG. 6 is a schematic view showing a plasmid name: pUC118-LDH KO-GenR (plasmid number: KCP0102).
FIG. 7 is a schematic view showing a plasmid name: pUC118-LDH KO-trc-lldD-GenR (plasmid number: KCP0100).
FIG. 8 is a schematic view showing a plasmid name: pUC118-LDH KO-trc-lldD-lldP-GenR (plasmid number: KCP0101).
FIG. 9 is a view showing a cell proliferation test result of an llD/lldP introduced strain according to an embodiment of the present disclosure. A graph showing cell proliferation of each test strain (FIG. 9, upper side) and a culture photograph at each point (FIG. 9, lower side) are shown.
FIG. 10 is a graph showing consumption of nitrate and phosphate of the llD/lldP introduced strain according to an embodiment of the present disclosure. The consumption of nitrate is illustrated on the upper side of FIG. 10, and the consumption of phosphate is illustrated on the lower side of FIG. 10.
FIG. 11 is a graph showing consumption of lactate of the llD/lldP introduced strain according to an embodiment of the present disclosure.
FIG. 12 is a schematic view showing analysis results of the amounts of intracellular and extracellular metabolites of the llD/lldP introduced strain according to an embodiment of the present disclosure.
FIG. 13 is a schematic view showing analysis results of the amount of TCA cycle metabolites of the llD/lldP introduced strain according to an embodiment of the present disclosure.
FIG. 14 is a schematic view showing analysis results of the amounts of various amino acids in the llD/lldP introduced strain.
FIG. 15 is a schematic view showing sustainable and efficient food production through a cell culture using algae as a nutrient source and organization according to an embodiment of the present disclosure.
FIG. 16 is a schematic view showing a circular cell culture according to an embodiment of the present disclosure.
FIG. 17 is a schematic view showing a production process of cultured meat according to an embodiment of the present disclosure.

### Best Mode for Carrying Out the Invention

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

In the present specification, "about" means ±10% of its subsequent numerical value.

In the present specification, "utilization ability" or "usability" refers to an ability of an organism to uptake a substance into a cell and metabolize or consume it, and can be used interchangeably.

In the present specification, "imparting or enhancing" the utilization ability refers to imparting utilization ability to an organism by genetically modifying the organism in a case where the organism does not originally have the utilization ability, or to improving the utilization ability by genetically modifying the organism in a case where the organism originally has the utilization ability.

In the present specification, "L-lactate utilization ability" or "L-lactate usability" refers to an ability to uptake L-lactate into a cell and metabolize or consume it, and can be used interchangeably.

In the present specification, the "lactate derivative or the derivative thereof refers to a compound produced by converting lactate in an organism or a cell, or a compound produced by modifying or processing the compound.

In the present specification, the "modification" refers to insertion of a nucleic acid sequence into DNA in a cell, partial deletion of a nucleic acid sequence of DNA in a cell, or a combination thereof.

In the present specification, the "organism which undergoes a modification" or the "modified organism" refers to an organism in which a nucleic acid sequence of DNA in an organism is modified compared to the reference organism. Examples of the reference organism include, but are not limited to, an organism that exists in nature, an organism that was unknown before a modification, and an organism that did not exist before a modification.

In the present specification, the "gene" refers to a factor that defines a genetic trait, and the "gene" may refer to "polynucleotide", "oligonucleotide", or "DNA". Such a gene encoding a protein may be endogenous or exogenous in an organism to be targeted. In addition, these known genes can be appropriately used. The gene can be used regardless of its origin. That is, the gene may be derived from an organism of another species or an organism of another genus other than a target organism, or may be derived from an organism such as an animal, a plant, a fungus (mold or the like), or a bacterium. Those skilled in the art can appropriately obtain such information on genes by accessing HP such as National Center for Biotechnology Information (NCBI, http://www.ncbi.nlm.nih.gov). These genes may be genes encoding proteins having a certain relationship with sequence information disclosed in a database or the like as long as they have each activity. Examples of such an aspect include a gene that consists of an amino acid sequence in which one or a plurality of amino acids are deleted, substituted, inserted, or added in the disclosed amino acid sequence and encodes a protein having an activity to be enhanced in the present invention. A mutation of the amino acid to the disclosed amino acid sequence, that is, the deletion, substitution, insertion, or addition may be any one kind, or a combination of two or more kinds. In addition, the total number of these mutations is not particularly limited, and is preferably about 1 or more and 10 or less. More preferably, the total number of these mutations is 1 or more and 5 or less. The amino acid substitution is preferably a preservative substitution, and specifically, examples thereof include substitutions within the following groups. (Glycine and alanine) (valine, isoleucine, and leucine) (aspartic acid and glutamic acid) (asparagine and glutamine) (serine and threonine) (lysine and arginine) (phenylalanine and tyrosine).

In the present specification, the "introduction" of a gene refers to introduction of an exogenous or endogenous gene, and preferably a functional gene, into, for example, a chromosomal genome or the like by an appropriate introduction technique. For the gene introduction, a gene can be introduced using a vector such as a phage or a plasmid, and a natural transformation method, a conjugation method, a protoplast-PEG method, an electroporation method, or the like can also be used. In addition, when a target gene recombination method known in the art is used, an exogenous functional gene can be introduced by substituting with an endogenous functional gene. Note that the exogenous functional gene is a gene that is not originally present in the chromosomal genome of the organism, and may be a gene derived from another species or a synthetic gene produced by PCR or the like. The introduction of a gene also includes conversion into a desired gene by performing genome editing on an existing genome.

In the present specification, the "membrane permeability" refers to an ability of a compound to permeate a cell membrane, and includes the fact that the compound is partially embedded in the cell membrane and the fact that the compound completely passes through the cell membrane to reach an intracellular space. Preferably, in the context of the present disclosure, it is advantageous that a foreign substance (for example, L-lactate or the like) permeates from the outside to the inside in an assimilable form.

In the present specification, the "gene that performs synthesis" of L-lactate or D-lactate refers to a gene encoding an enzyme or the like involved in promoting L-lactate synthesis or D-lactate synthesis in a living body or in a cell, or a gene that promotes expression of an enzyme or the like involved in promoting L-lactate synthesis or D-lactate synthesis. For example, when pyruvic acid is a substrate, D-lactate dehydrogenase (EC 1.1.1.28) corresponds to the gene (product) that performs the synthesis.

In the present specification, the "attenuation" of a gene refers to reducing gene expression (may be at a transcription level or at a translation level) or a function of a gene product compared to the natural state or before modification, and the "deletion" of a gene refers to deleting gene expression (may be at a transcription level or at a translation level) or a function of a gene product. In the present specification, the "attenuation" and "deletion" of a "gene" can be described as "attenuation" and "deletion" of the nucleic acid in which the gene is encoded and the product thereof.

In the present specification, the "circulation" and "recycle" mean that in a certain system, a certain substance or an organic body composed of a substance, and preferably all substances present in a certain systems or organic bodies composed of substances are reused without being supplied from the outside in the system, and a target substance or an organic body composed of a substance is reproduced. For example, a case where a second organism or a cultured cell uses a component and the like excreted from a first organism or a cultured cell as a nutrient source, and a first organism or a cultured cell uses a component and the like excreted from a second organism or a cultured cell as a nutrient source corresponds to circulation and recycle.

In the present specification, the "excreted component" and "a component to be excreted" refer to a component excreted from the inside of a cell to the outside in a step of culturing cells, the excretion can take any form such as simple elimination of waste products, evaporation, and diffusion in addition to secretion, and the component also includes a component (for example, L-lactate or the like for a certain animal cell) toxic to the cell.

In the present specification, the "nutritional component" and "nutrient source" are used interchangeably, and refer to a component that is consumed or metabolized when an organism or a cultured cell grows or proliferates, and include, for example, a sugar, an amino acid, or the like, or a complex thereof.

In the present specification, the "a component derived from" refer to an organism or a cultured cell itself, a part thereof, or a component produced by the organism or the cultured cell.

In the present specification, the "product" refers to a substance produced or the like by an organism or a cultured cell, and includes, for example, an amino acid, a protein, a saccharide, and the like.

In the present specification, the "cultured in different places" refers to culturing in a spatially or physically distant place.

In the present specification, the "three-dimensional organization" means that the amplified cells are organized into a spherical shape, a thread-like shape, or a layered shape, and further integrated to have a constant three-dimensional size.

In the present specification, the "pyruvic acid metabolism system" means a system that converts pyruvic acid into another compound in an organism or a cell. Metabolism also includes uptaking pyruvic acid into an organism or a cell, and producing various compounds using the uptaken pyruvic acid as a material. Pyruvic acid (pyroracemic acid) is produced from phosphoenolpyruvate in a glycolytic system, becomes acetyl-CoA, and is then incorporated into a TCA cycle, but it also serves as a raw material for the metabolic production of various other compounds. Pyruvic acid or metabolites thereof include, in addition to a substance involved in a pyruvic acid metabolism system, any sugar, organic acid, or substance such as an amino acid, and a secondary metabolite and a derivative thereof that can be obtained by altering and/or modifying a specific substance in the system. Therefore, examples of the metabolite of pyruvic acid include amino acids such as glutamine, glutamic acid, alanine, valine, and leucine, or D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, and butane.

In the present specification, the "TCA cycle" is a biochemical reaction circle related to aerobic metabolism, and is found in all organisms that perform aerobic respiration. Acetyl CoA generated by glycolysis or β-oxidation of fatty acid is incorporated into the circle and oxidized, such that NADH and the like used in ATP or an electron transport system can be produced, which makes it possible to produce energy. In addition, a biosynthetic precursor such as an amino acid is provided. The TCA cycle may also be referred to as a TCA cycle, a tricarboxylic acid circle, a citric acid circle, a Krebs cycle, or the like.

In the present specification, the "synthetic pathway for succinic acid, 2-oxoglutaric acid, 3-hydroxypropionic acid, acrylic acid, and/or acetic acid" refers to a pathway in which succinic acid, 2-oxoglutaric acid, 3-hydroxypropionic acid, acrylic acid, and/or acetic acid is synthesized from another compound in an organism or a cell.

Examples of the metabolic pathway of the amino acid include the following. An aromatic amino acid is synthesized via a shikimate pathway. In the shikimate pathway, colismic acid is synthesized starting from a reaction that condenses phosphoenolpyruvate (PEP) and erythrose 4-phosphate (E4P) to synthesize 3-deoxy-D-arabino-heptulosonic acid 7-phosphate (DAHP). Then, phenylalanine, tyrosine, and tryptophan as aromatic amino acids are synthesized using colismic acid as a substrate. When the shikimate pathway is controlled, all aromatic amino acids and secondary metabolites involved in the pathway are also controlled. An enzyme group of the aromatic amino acid synthetic pathway catalyzes the biosynthesis of aromatic amino acids from erythrose 4-phosphate (E4P). The aromatic amino acid synthetic pathway includes an enzyme group that biosynthesizes aromatic amino acids such as tyrosine (Tyr), phenylalanine (Phe), and tryptophan (Trp) from erythrose 4-phosphate (E4P) biosynthesized in the pentose phosphate pathway via colismic acid. In addition, pyruvate carboxylase (for example, PYC1, PYC2, or the like) is an enzyme that biosynthesizes oxaloacetic acid (OAA) from pyruvic acid biosynthesized in a glycolytic system. Pyruvate dehydrogenase (PDA1) is a complex enzyme that synthesizes acetyl CoAfrom pyruvic acid biosynthesized in a glycolytic system. In general, although ARO1, ARO2, ARO3, ARO4, ARO7, ARO8, ARO9, TYR1, PHA2, TRP2, TRP3, TRP4, TRP1, TRP3, TRP5, and the like are present in aromatic amino acid synthetic pathway genes, ARO1, ARO7, ARO8, ARO9, PHA2, TRP5, and the like are preferably used as enhancement targets.

The pathways related to synthesis or metabolism of succinic acid, 2-oxoglutaric acid, 3-hydroxypropionic acid, acrylic acid, and/or acetic acid, enzymes that catalyze the pathways, and the like are exemplified in, for example, Nature Catalysis 2, 18-33 (2019).

In addition, pathways related to synthesis or metabolism of an amino acid, enzymes that catalyze these pathways, and the like are exemplified in, for example, Eukaryot Cell. 2006 Feb.; 5 (2): 272-276, Current Opinion in Microbiology 32, 151-158 (2016), Annual Review of Plant Biology 67, 153-78 (2016), Pest Management Science 76(12), 3896-3904 (2020), and the like.

### (Preferred Embodiments)

Preferred embodiments of the present disclosure will be described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description in the present specification. In addition, the following embodiments of the present disclosure can be used alone or in combination.

### (Modified Organism)

In one aspect of the present disclosure, an organism or a cultured cell which undergoes a modification is provided, in which the modification includes imparting or enhancing L-lactate utilization ability in the organism or the cultured cell. L-Lactate is one of the main a component of an animal cell culture waste liquid, and in an embodiment of the present disclosure, such a modified organism or cultured cell is provided, such that the modified organism or cultured cell can be grown by utilizing L-lactate, and a component derived from the modified organism or cultured cell can be used as a nutrient source for animal cultured cells.

In the construction of a circulation food production system using organisms such as microalgae and blue-green algae and animal cultured cells, it is required for organisms such as microalgae and blue-green algae to utilize L-lactate, which is a main a component excreted from animal cultured cells. However, a gene encoding a converting enzyme of L-lactate has not been found in many microalgae and blue-green algae, and L-lactate utilization by microalgae and blue-green algae has not been reported so far. Indeed, preliminary experiments are conducted on some microalgae and blue-green algae (Chlamydomonas sp. KOR1, Pavlova sp. OPMS 30543, Arthrospira platensis NIES-39, Anabaena sp. PCC 7120, and Synechococcus sp. PCC 7002), and it is confirmed that they do not consume L-lactate. The list of the presence or absence of NAD-independent L-lactate dehydrogenase (L-iLDH) and lactate 2-monooxygenase (LMO) in microalgae and blue-green algae is as follows. In the table, the species indicated by the black circles are species having L-iLDH or LMO, and can also be used in Examples of the present disclosure as an example.

As the blue-green algae, various kinds of blue-green algae can be used. The blue-green algae (cyanobacteria) are typically unicellular prokaryotes and perform oxygen-generating type photosynthesis, and can be classified into the order Chroococcales, the order Nostocales, the order Oscillatoriales, the order Pleurocapsales, the order Stigonematales, and the like. Examples of the order Chroococcales include the genus Aphanocapsa, the genus Aphanothece, the genus Chamaesiphon, the genus Chroococcus, the genus Crocosphaera, the genus Cyanobacteria, the genus Cyanobium, the genus Cyanothece, the genus Dactylococcopsis, the genus Gloeobacter, the genus Gloeocapsa, the genus Gloeothece, the genus Euhalothece, the genus Halothece, the genus Johannesbaptistia, the genus Merismopedia, the genus Microcystis, the genus Rhabdoderma, the genus Synechococcus, the genus Synechocystis, and the genus Thermosynechococcus. Examples of the order Nostocales include the genus Coleodesmium, the genus Fremyella, the genus Microchaete, the genus Rexia, the genus Spirirestis, the genus Tolypothrix, the genus Anabaena, the genus Anabaenopsis, the genus Aphanizomenon, the genus Aulosira, the genus Cyanospira, the genus Cylindrospermopsis, the genus Cylindrospermum, the genus Nodularia, the genus Nostoc, the genus Richelia, the genus Calothrix, the genus Gloeotrichia, and the genus Scytonema. Examples of the order Oscillatoriales include the genus Arthrospira, the genus Geitlerinema, the genus Halomicronema, the genus Halospirulina, the genus Katagnymene, the genus Leptolyngbya, the genus Limnothrix, the genus Lyngbya, the genus Microcoleus, the genus Oscillatoria, the genus Phormidium, the genus Planktothricoides, the genus Planktothrix, the genus Plectonema, the genus Limnothrix, the genus Pseudanabaena, the genus Schizothrix, the genus Spirulina, the genus Symploca, the genus Trichodesmium, and the genus Tychonema. Examples of the order Pleurocapsales include the genus Chroococcidiopsis, the genus Dermocarpa, the genus Dermocarpella, the genus Myxosarcina, the genus Pleurocapsa, the genus Stanieria, and the genus Xenococcus. Examples of the order Stigonematales include the genus Capsosira, the genus Chlorogloeopsis, the genus Fischerella, the genus Hapalosiphon, the genus Mastigocladopsis, the genus Mastigocladus, the genus Nostochopsis, the genus Stigonema, the genus Symphyonema, the genus Symphyonemopsis, the genus Umezakia, and the genus Westiellopsis.

In addition, the "microalgae" are typically unicellular eukaryotes that perform oxygen-generating type photosynthesis, and in the present disclosure, the "microalgae" also include secondary symbiotic algae among unicellular eukaryotes that do not perform photosynthesis. Examples of the microalgae to be applied include, but are not limited to, algae of euglena (euglena algae or the like), algae of the genus Chlamydomonas, algae of the genus Chlorella, algae of the genus Botryococcus, blue-green algae of the genus Aphanothece (Aphanothece sacrum or the like), or blue-green algae of the genus Oscillatoria (spirulina or the like).

Therefore, in an embodiment of the present disclosure, an L-lactate dehydrogenase gene and/or a lactate permease gene can be introduced into an organism such as blue-green algae to impart L-lactate usability.

In an embodiment, the organism or the cultured cell to be modified is not particularly limited as long as it is a species that can be modified to impart or enhance L-lactate usability. For example, examples of the organism or the cultured cell to be modified include blue-green algae, microalgae, a yeast, E. coli, Bacillus subtilis, corynebacterium, actinomycetes, filamentous fungus, Bacillus subtilis, Lactobacillus, and cultured cells of plants and animals. In an embodiment of the present disclosure, examples of the organism or the cultured cell to be modified include an organism or a cultured cell that can be used in the resource circulation food production system described below, and in this case, the organism or the cultured cell to be modified is preferably an organism or a cultured cell that can immobilize carbon dioxide by photosynthesis and supply glucose as a nutrient source to animal cells.

In an embodiment of the present disclosure, the modification performed on the organism or the cultured cell as described above is not particularly limited as long as it imparts or enhances the L-lactate utilization ability, and includes modification, introduction, and/or deletion of a gene. For example, in an embodiment, a gene that imparts or enhances usability or membrane permeability of L-lactate to the organism or cultured cell described above can also be introduced. In addition, in another embodiment, the gene for synthesis of L-lactate or D-lactate can also be attenuated or deleted.

Examples of the gene that utilizes L-lactate include a gene encoding NAD-independent L-lactate dehydrogenase (EC number 1.1.2.3) such as lldD of E. coli, a gene encoding lactate 2-monooxygenase (EC 1.13.12.4) of Mycolicibacterium smegmatis and the like, and a gene encoding lactate oxidase (EC number 1.1.3.2) of Aerococcus viridans and the like. Since it has been reported that NAD-independent L-lactate dehydrogenase (EC number 1.1.2.3) contributes to the use of L-lactate in addition to lldD of E. coli (for example, Corynebacterium glutamicum (lldD gene) (Appl Environ Microbiol. 2005 Oct; 71(10): 5920-5928), Pseudomonas aeruginosa (lldAgene and lldD gene) (Environ Microbiol Rep. 2018 Oct; 10(5): 569-575), Pseudomonas stutzeri (lldABC gene) (J Bacteriol. 2015 Jul; 197(13): 2239-2247), Clostridium acetobutylicum (Archives of Microbiology volume 164, pages 36-42 (1995)), and the like), in the present disclosure, L-lactate utilization ability can be imparted to blue-green algae by introduction of lldD of E. coli, and therefore, L-lactate utilization ability can be similarly imparted not only from E. coli but also using the same type of enzyme. In addition, it has been reported by a large number of documents that various foreign genes including those derived from E. coli function in blue-green algae. For example, as an example in which the production of a compound that is not originally produced by the blue-green algae is successfully performed by introduction of a foreign gene, in ACS Synth Biol. 2019 Dec 20; 8(12): 2701-2709, astaxanthin was successfully produced by introducing a β-carotene ketolase gene and a β-carotene hydroxylase gene derived from Brevundimonas sp. into the cyanobacterium Synechococcus sp. PCC 7002, in Front Bioeng Biotechnol. 2014 Jun 19; 2: 21, limonene and bisabolene were successfully produced by introducing an L-limonene synthase gene derived from Mentha spicata and an (E)-α-bisabolene synthase gene derived from Abies grandis into the cyanobacterium Synechococcus sp. PCC 7002, respectively, in Front Bioeng Biotechnol. 2015 Apr 24; 3: 48, lauric acid was successfully produced by introducing a lauroyl-acyl carrier protein thioesterase gene derived from Umbellularia californicay into cyanobacterium Synechococcus sp. PCC 7002, and in Biosci Biotechnol Biochem. 2013; 77(5): 966-70, L-lactate was successfully produced by introducing L-lactate dehydrogenase derived from Lactococcus lactis, Lactobacillus plantarum, and Lactobacillus rhamnosus into cyanobacterium Synechocystis sp. PCC 6803. In addition, in Metab Eng. 2020 Jan; 57: 129-139, an aromatic compound that is not originally produced by PCC 6803 was successfully produced by enhancing production of an aromatic amino acid through introduction of aroGfbr and tyrAfbr genes derived from E. coli and further introducing a tyrosine/phenylalanine ammonia lyase gene derived from Trichosporon cutaneum and a 4-hydroxyphenlacetate 3-hydroxylase complex derived from E. coli into cyanobacterium Synechocystis sp. PCC 6803. In addition, in Microb Cell Fact. 2017 Feb 23; 16(1): 34, ethylene was successfully produced by introducing an ethylene-forming enzyme gene derived from Pseudomonas syringae into cyanobacterium Synechocystis sp. PCC 6803. Therefore, in the present disclosure, it is considered that foreign genes other than E. coli can also be used for imparting L-lactate utilization ability to blue-green algae.

Examples of the gene that imparts or enhances the membrane permeability of L-lactate include a gene encoding a lactate permease such as lldP of E. coli, and a gene encoding a monocarboxylate transporter.

Examples of the gene for synthesizing L-lactate or D-lactate include a gene encoding an NAD-dependent L-lactate acid dehydrogenase (EC 1.1.1.27), a gene encoding a lactaldehyde dehydrogenase (EC 1.2.1.22), a gene encoding a malolactic enzyme (EC 4.1.1.101), and a gene encoding a D-lactate dehydrogenase (EC 1.1.1.28) such as ldhA of blue-green algae.

In an embodiment, L-lactate usability is imparted and enhanced for an organism or a cultured cell to be modified, such that a nutrient source of the animal cultured cells can be provided using a component excreted from the animal cultured cells. Therefore, in an embodiment of the present disclosure, the organism or the cultured cell of the present disclosure can have a pyruvic acid metabolism system and/or a TCA cycle. In addition, in an embodiment of the present disclosure, the organism or the cultured cell of the present disclosure has a synthetic pathway of succinic acid, 2-oxoglutaric acid, 3-hydroxypropionic acid, acrylic acid, and/or acetic acid.

In an embodiment of the present disclosure, the organism or the cultured cell to be modified can also utilize a component excreted from the animal cultured cells to perform useful amino acid synthesis, and thus can also be a source of amino acids. Therefore, in an embodiment of the present disclosure, the organism or the cultured cell of the present disclosure can have a synthetase and/or a synthetic system for an amino acid such as glutamine, glutamic acid, alanine, valine, or leucine, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane, etc. These synthetases and synthetic systems are exemplified, for example, in Nature Catalysis 2, 18-33 (2019).

### (Resource Circulation Food Production System)

In the present food production system, after breeding individual animals and plants, such as cultivating rice and harvesting rice or raising livestock and collecting meat, some of the animals and plants are used as food. Therefore, a part other than an edible part of the individual is discarded, resulting in a lot of waste. In addition, since in plant cultivation, a large amount of agricultural chemicals and fertilizers are used to consume a large amount of resources and energy, and soil is contaminated, in livestock breeding, accumulation of greenhouse gases such as methane gas emitted is an environmental load.

In recent years, in order to solve these problems, an attempt to produce food from cells mainly including cultured meat (cell agriculture) has been started in the world. However, nutrients contained in a medium required for culturing animal cells are originally derived from plants such as grains. In addition, measures against the resulting culture waste liquid are also insufficient. In the future, with the spread of cell agriculture, it is predicted that a large amount of medium will be used as a nutrient source for cell culture, a large amount of culture waste liquid will also be generated, and there is a risk that a new environmental load will be caused. Therefore, an embodiment of the present disclosure provides a waste-free production system in a process of cell agriculture.

Therefore, in an aspect of the present disclosure, there is provided a method for culturing at least two types of cells including a cell X and a cell Y in a circulation manner, the method including: a step (a) of providing a component excreted from the cell X to the cell Y; a step (b) of providing a component derived from the cell Y to the cell X; a step (c) of culturing the cell X and the cell Y; and a step (d) of repeating the steps (a) to (c) as necessary, in which at least one of the component excreted from the cell X is an assimilable a component of the cell Y, and at least one of the component derived from the cell Y is a nutritional a component of the cell X. In an embodiment of the present disclosure, by such a method, it is possible to provide a waste-free, environmentally friendly, and healthy circulation culture food production system that amplifies only cells of edible part tissues of plants and animals and simultaneously reduces problems of nutrient source supply and a culture waste liquid.

In an embodiment, it is also possible to provide a method for producing a desired product by using, as at least one of the cell X or Y, a cell that produces a desired product in the method as described above.

In an embodiment of the present disclosure, examples of the cell X include animal cells, and for example, cells that can be subjected to edible meat production by culture are preferable. In addition, in an embodiment, a component derived from the cell Y can also be used as a nutrient source of the cell X directly or indirectly by performing a catalytic treatment or the like. For example, the cell Y can include blue-green algae, microalgae, a yeast, E. coli, Bacillus subtilis, corynebacterium, actinomycetes, filamentous fungus, Bacillus subtilis, and Lactobacillus. The cells X and Y may be the same type of cells as long as they complement each other.

In addition, in an embodiment, a component excreted from the cell X is not particularly limited as long as it can utilize the cell Y, and can include, for example, L-lactate, carbon dioxide, ammonium, urea, or phosphate. In addition, in an embodiment, a component derived from the cell Y is not particularly limited as long as they can serve as nutritional a component of the cell X, and examples thereof include glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B 1, vitamin B2, vitamin B3, vitamin B5, vitamin B6, folate, choline, starch, glycogen, and cellulose. In an embodiment of the present disclosure, a component excreted from the cell X and a component derived from the cell Y are not necessarily essential nutritional a component for the other cells, and may be arbitrary a component that can be utilized. For example, L-lactate excreted from animal cells is not an essential nutritional a component for blue-green algae, but can be utilized by modified blue-green algae. Similarly, ammonium discharged from animal cells is a compound that can be utilized by blue-green algae, and is essential for the blue-green algae when there is no compound serving as a nitrogen source other than the compound, or when the blue-green algae having a nitrogen immobilization ability are not used.

In an embodiment, the cells X and Y can be cultured to obtain a circulation culture method by using a component excreted from one cell or a component derived from the cell as a nutrient source of the other cell. In this case, the cells X and Y may be cultured in the same place or may be cultured in different places.

In addition, in another aspect of the present disclosure, there is provided a method for culturing animal cells by circulating a component excreted from an animal cell culture by an organism, the method including: a step (a) of causing an organism to consume a component to be excreted, the organism having utilization ability of the component; a step (b) of recovering a nutrient source of the animal cells from a culture solution of the organism; a step (c) of culturing the animal cells using the nutrient source; and a step (d) of repeating the steps (a) to (c) as necessary.

In an embodiment of the present disclosure, it is possible to provide a circulation cell culture system having high efficiency and low environmental load in which algae capable of synthesizing organic matter from inorganic matter by photosynthesis are cultured to circulate substances such as carbon and nitrogen. In addition, it is possible to provide a three-dimensional organization system that produces only edible part tissues by amplification of animal cells. In such a circulation system, it is possible to realize amplification of animal cells by algae-derived nutrients and circulating of a culture waste liquid by utilizing cell culture engineering, catalytic chemistry, and genetic engineering. In addition, in an embodiment of the present disclosure, the method of the present disclosure can reduce the waste of the conventional culture food production technique. Tissue engineering in the field of regenerative medicine can also be applied and developed to a three-dimensional organization system from cells, and it is possible to realize a bioeconomical culture food production system using cells as food materials by integrating both systems.

In an embodiment of the present disclosure, as a nutrient source of animal cells, a component that utilizes and excretes a component excreted from animal cells by an organism, or a component constituting the organism itself can also be directly or indirectly used as a nutrient source by performing a catalytic treatment or the like. Examples of the nutrient source of the animal cells includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B 1, vitamin B2, vitamin B3, vitamin B5, vitamin B6, folate, and choline. In addition, in another embodiment, it is also possible to hydrolyze carbohydrates (starch glycogen) accumulated in cells of microalgae and blue-green algae and cellulose of a cell wall, and use glucose obtained thereby as a nutrient source of animal cells.

In an embodiment of the present disclosure, a component excreted from the animal cell culture may be arbitrary a component that can be utilized and consumed by an organism and used for production of a nutrient source to be supplied to animal cells, and examples thereof include L-lactate, carbon dioxide, ammonium, urea, and phosphate. In an embodiment, a component excreted from the animal cell culture may be toxic to the animal cells, and examples thereof include L-lactate, ammonium, and urea.

In an embodiment of the present disclosure, it is possible to provide a system capable of culturing algae using light energy as a drive source, supplying nutrients to animal and plant cells by decomposition of algae, culturing animal and plant cells serving as food materials, and circulating a culture waste liquid. Further, a three-dimensional organization system for forming a three-dimensional tissue sufficient to eat the amplified cells is provided, such that it is possible to provide a circulation culture food production system using the cells as food materials.

In an embodiment, in the method of the present disclosure, it is required to realize, at the cell level, the action at the individual level that the animal eats the plant. Therefore, in an embodiment, the method of the present disclosure can decompose organic matter synthesized by algae to each nutrient that can be used by animal and plant cells by using a physical (ultrasonic), chemical (solid acid), or biological (decomposing enzyme gene transfer) technique. The nutrient thus obtained can be used as a medium component.

In an embodiment, in the method of the present disclosure, in order to construct a highly efficient cell amplification process that maximizes a cell function, it is required to amplify cells without externally adding bovine serum, growth factors, and/or hormones that are usually used for cell culture. Therefore, in an embodiment, the method of the present disclosure can culture a plurality of cells such as cells originally having an ability to secrete a growth factor or a hormone in a living body or cells into which a corresponding gene is introduced, and can culture a target cell to be a food material using the culture supernatant.

In an embodiment, in the method of the present disclosure, since a large amount of culture waste liquid generated in cell culture leads to an environmental load, efficient circulating of substances contained in the culture waste liquid is required. Therefore, in an embodiment, the method of the present disclosure can also perform comprehensive a component analysis of the culture waste liquid and selection of algae using these substances, and can perform waste liquid circulating in which useful a component obtained by separating and selecting the culture waste liquid directly or by various methods are used as a nutrient source of algae. In an embodiment, some a component derived from waste liquid can also be used in cell culture of animals and plants.

In an embodiment, in the method of the present disclosure, it is required to link the processes as described above, stably operate the processes, and improve efficiency. Therefore, in an embodiment, the method of the present disclosure can also provide a stable and efficient system in which parameters that are important in each culture process are selected and a monitoring technique and control thereof are incorporated.

In an embodiment, in the method of the present disclosure, it is required to realize organization equivalent to the current food from the cell alone in terms of nutrition and texture. Therefore, in an embodiment, the method of the present disclosure can provide a production system in which, for animal cells, a three-dimensional tissue is constructed from cells by making full use of tissue engineering that maximizes a self-organizing ability and is developed in regenerative medical research, and, for example, a cultured sashimi, a cultured chicken fillet, or the like is formed. In an embodiment, since plant cells originally have a self-tissue forming ability from a single cell (callus cell), it is also possible to provide a production system for forming cultured rice, cultured root vegetables, and cultured pulp by establishing a technique for controlling culturing of callus using nutrients derived from algae or animal cells.

In an embodiment, in the method of the present disclosure, it is required to stably and safely produce healthy and delicious food sustainably and efficiently. Therefore, in an embodiment, the method of the present disclosure can provide a bioeconomical cultured food production system by aseptically integrating a series of systems, monitoring and controlling a production process, and optimizing quality and efficiency.

In an embodiment, in the method of the present disclosure, an organism or a cultured cell that produces glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B1, vitamin B2, vitamin B3, vitamin B5, vitamin B6, folate, choline, starch, glycogen, or cellulose can be used. In such an organism or cultured cell, these compounds, or enzymes for synthesis or metabolism of them may be contained, or modifications may be made so that such synthesis or metabolism can be performed. Pathways related to amino acid synthesis or metabolism, enzymes that catalyze these pathways, and the like are exemplified in, for example, Eukaryotic Cell. 2006 Feb.; 5 (2): 272-276, Current Opinion in Microbiology 32, 151-158 (2016), Annual Review of Plant Biology 67, 153-78 (2016), Pest Management Science 76(12), 3896-3904 (2020), and the like, and in an embodiment, the organism or the cultured cell may be modified to be able to synthesize or metabolize them.

In addition, the synthesis of glutamic acid from L-lactate in blue-green algae converts L-lactate into pyruvic acid by NAD-independent L-lactate dehydrogenase, and pyruvic acid to glutamic acid. Such pathways are exemplified, for example, in PLoS One 9(7), e103380 (2014) and the like, and in an embodiment, the organism or the cultured cell may be modified so as to be enable synthesis or metabolism for them to be performed.

In an embodiment of the present disclosure, the production of the cultured meat can include the following steps. Myocytes are collected from livestock muscle tissue, the myocytes are amplified in a large amount in vitro, the amplified cells are three-dimensionally organized by a tissue engineering technique, and then the prepared three-dimensional tissue is matured by tissue culture. Techniques for three-dimensional organization of amplified cells include a method in which a material to be a cell scaffold is used and cells are seeded on the scaffold (EMBO reports 2019; 20: e47395, NPJ Sci Food 2021; 5: 6, ACS Appl Mater Interfaces 2021; 13: 32193-32204) or a method using a spheroid technique (Biofabrication 2021; 13. doi: 10.1088/1758-5090/ac23e3) and a cell sheet technique (Circ Res 2002; 90: e40-48).

In addition, in another aspect of the present disclosure, there is provided a method for producing cultured meat by circulating a component excreted from an animal cell culture by an organism and culturing animal cells, the method including: a step (a) of causing an organism to consume a component to be excreted, the organism having utilization ability of the component; a step (b) of recovering a nutrient source of the animal cells from the organism; a step (c) of culturing the animal cells using the nutrient source; a step (d) of repeating the steps (a) to (c) as necessary; and a step (e) of subjecting the cultured animal cells to three-dimensional organization and molding the three-dimensionally organized animal cells into cultured meat. In an embodiment, also in the method for producing cultured meat, the embodiments described in other parts of the present specification can be appropriately selected and used.

In addition, in another aspect of the present disclosure, there is provided a method for producing an amino acid and/or a desired product in an organism or a cultured cell using L-lactate excreted from an animal cell culture, the method including: a step of providing the L-lactate to the organism or the cultured cell, the organism or the cultured cell having L-lactate utilization ability or being modified to impart or enhance L-lactate utilization ability in the organism or the cultured cell; a step of culturing the organism or the cultured cell under conditions in which L-lactate is utilized; and a step of recovering the amino acid as necessary. In an embodiment, also in the method for producing an amino acid and/or a desired product in an organism or a cultured cell, the embodiments described in other parts of the present specification can be appropriately selected and used.

In addition, in another aspect of the present disclosure, there is provided a method for producing a processed product of an amino acid and/or a desired product produced by an organism or a cultured cell using L-lactate excreted from an animal cell culture, the method including: a step of providing the L-lactate to the organism or the cultured cell, the organism or the cultured cell having L-lactate utilization ability or being modified to impart or enhance L-lactate utilization ability in the organism or the cultured cell; a step of culturing the organism or the cultured cell under conditions in which L-lactate is utilized; a step of recovering the amino acid and/or the desired product; and a step of processing the amino acid and/or the desired product. In an embodiment, also in the method for producing a processed product, the embodiments described in other parts of the present specification can be appropriately selected and used.

In addition, in another aspect of the present disclosure, there is provided a method for breeding an organism or a cultured cell using a component excreted from an animal cell culture, the method including: a step (A) of providing a component excreted from animal cells to a candidate organism or cultured cell; a step (B) of determining whether the organism or the cultured cell consumes the excreted a component or enhances the consumption; and a step (C) of selecting an organism or a cultured cell in which consumption is imparted or enhanced among the organisms or cultured cells, in which the selected organism or cultured cell is determined to have utilization ability of a component or to be modified to impart or enhance utilization ability of a component in the organism or the cultured cell. In an embodiment, also in the method for breeding an organism or a cultured cell, the embodiments described in other parts of the present specification can be appropriately selected and used.

In addition, in another aspect of the present disclosure, there is provided a method for maintaining an organism or a cultured cell using a component excreted from an animal cell culture, the method including: a step (a) of causing the organism or the cultured cell to consume a component to be excreted, the organism or the cultured cell having utilization ability of a component or being modified to impart or enhance utilization ability of a component in the organism or the cultured cell; a step (b) of recovering a nutrient source of the animal cells from the organism or the cultured cell as necessary; a step (c) of culturing the animal cells using the nutrient source as necessary; and a step (d) of providing a component excreted from the animal cells to the organism or the cultured cell. In an embodiment, also in the method for maintaining an organism or a cultured cell, the embodiments described in other parts of the present specification can be appropriately selected and used.

In addition, in another aspect of the present disclosure, there are provided a gene used for breeding a modified organism or cultured cell described in other parts of the present specification, or a kit containing the gene. In an embodiment, also in such a kit, the embodiments described in other parts of the present specification can be appropriately selected and used.

In addition, in another aspect of the present disclosure, there is provided a method for evaluating a modified strain, the method including: a step of preparing the organism or the cultured cell described in other parts of the present specification; and a step of confirming the presence of the modification in the organism or the cultured cell. In an embodiment, also in the method for evaluating a modified strain, the embodiments described in other parts of the present specification can be appropriately selected and used.

In addition, in another aspect of the present disclosure, there is provided a method for producing a plastic raw material from an organism or a cultured cell using L-lactate excreted from an animal cell culture, the method including: a step of providing the L-lactate to the organism or the cultured cell, the organism or the cultured cell having L-lactate utilization ability or being modified to impart or enhance L-lactate utilization ability in the organism or the cultured cell; a step of culturing the organism or the cultured cell under conditions in which L-lactate is utilized; and a step of recovering a component used as a plastic raw material from the organism or the cultured cell, in which the organism or the cultured cell is capable of synthesizing a component used as a plastic raw material, or a gene required for synthesis of a component used as a plastic raw material is introduced into the organism or the cultured cell. In an embodiment, also in the method for producing a plastic raw material from an organism or a cultured cell, the embodiments described in other parts of the present specification can be appropriately selected and used.

In addition, in another aspect of the present disclosure, there is provided a method for producing a lactate derivative or a derivative thereof, the method including: a step of providing L-lactate to an organism which undergoes a modification, the modification including imparting or enhancing L-lactate utilization ability in the organism; a step of culturing the organism under conditions in which L-lactate is utilized; and a step of recovering a desired lactate derivative or a derivative thereof as necessary. In an embodiment, also in the method for producing a lactate derivative or a derivative thereof, the embodiments described in other parts of the present specification can be appropriately selected and used.

In addition, in another aspect of the present disclosure, there is provided a method for producing pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid, the method including: a step of providing L-lactate to an organism which undergoes a modification, the modification including imparting or enhancing L-lactate utilization ability in the organism; a step of culturing the organism under conditions in which L-lactate is utilized; and a step of recovering a desired pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid as necessary. In an embodiment, also in the method for producing a lactate derivative or a derivative thereof, the embodiments described in other parts of the present specification can be appropriately selected and used.

In addition, in another aspect of the present disclosure, there is provided a method for producing a processed product of pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid, the method including: a step of providing L-lactate to an organism which undergoes a modification, the modification including imparting or enhancing L-lactate utilization ability in the organism; a step of culturing the organism under conditions in which L-lactate is utilized; a step of recovering a desired pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid as necessary; and a step of processing the pyruvic acid, acetyl CoA, phosphoenolpyruvate, succinic acid, 2-oxoglutaric acid, hydroxypropionic acid, acrylic acid, or acetic acid, or the derivative of pyruvic acid, acetyl CoA, phosphoenolpyruvate, succinic acid, 2-oxoglutaric acid, hydroxypropionic acid, acrylic acid, or acetic acid. In an embodiment, also in the method for producing a processed product of pyruvic acid, the embodiments described in other parts of the present specification can be appropriately selected and used.

### (General Technique)

The molecular biological technique, the biochemical technique, and the microbiological technique used in the present specification are well known and commonly used in the art, and are described in, for example, Sambrook J. et al. (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, the special volume of Experimental Medicine, "Experimental Methods for Gene Introduction & Expression Analysis", YODOSHA CO., LTD., 1997, and the like, and relevant parts (which may be all) of which are incorporated herein by reference.

For DNA synthesis techniques and nucleic acid chemistry for producing artificially synthesized genes, gene synthesis or fragment synthesis services such as GeneArt, GenScript, and Integrated DNA Technologies (IDT) can also be used, in addition, the DNA synthesis techniques and nucleic acid chemistry for producing artificially synthesized genes are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackbum, G.M. et al. (1996). Nucleic Acids in Chemistry and Biology, Oxford University Press; Hermanson, G.T. (I996). Bioconjugate Techniques, Academic Press, and the like, and relevant parts (which may be all) of which are incorporated herein by reference.

In the present specification, "or" is used when "at least one or more" of the items listed in the text can be employed. The same applies to "or". When explicitly described in the present specification as "within the range" of "two values", the range also includes the two values themselves.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present specification, but is limited only by the claims.

### Examples

In many microalgae and blue-green algae, genes encoding NAD-independent L-lactate dehydrogenase (EC 1.1.2.3) and lactate-2 monooxygenase (EC 1.13.12.4) have not been identified, and therefore, L-lactate cannot be utilized. The marine cyanobacterium Synechococcus sp. PCC 7002 has an excellent carbonic acid immobilization ability to immobilize CO₂ in the atmosphere by photosynthesis, and is a strain exhibiting high proliferation under photoautotrophic and seawater salt concentration conditions. Since it can be grown even with a salt concentration equivalent to that of the medium of animal cells, it is not required to adjust the salt concentration in utilizing the culture waste liquid, and it is promising in utilization of L-lactate derived from animal cells. However, in PCC 7002, a gene contributing to L-lactate utilization was not confirmed, and it was considered that PCC 7002 does not have L-lactate usability. Indeed, culturing PCC 7002 in a medium containing L-lactate did not consume L-lactate.

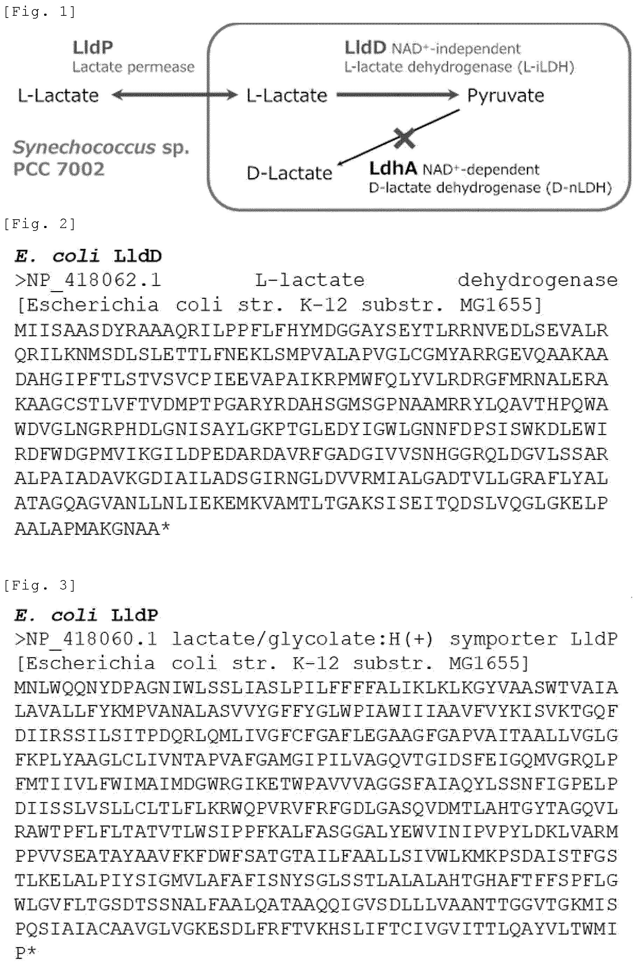

It was focused on that E. coli can grow using L-lactate as a single carbon source. By expressing an NAD-independent L-lactate dehydrogenase gene (lldD) and a lactate permease gene (lldP) constituting the lactate operon of E. coli, in the blue-green algae PCC 7002, it was attempted to convert L-lactate into pyruvic acid metabolizable by the blue-green algae in the blue-green algae cells (FIG. 1). At the same time, in order to prevent pyruvic acid derived from L-lactate from being utilized for synthesis of D-lactic acid, ldhA was disrupted by introducing an lldD/lldP gene into a D-lactate dehydrogenase gene (ldhA) site that contributes to synthesizing D-lactate of PCC 7002. Atrc promoter known to function in PCC 7002 was utilized for expression of the lldD/lldP gene.

### (Example 1: Construction of Vector for Introducing lldD/lldP)

A vector for introducing an E. coli-derived 11 dD/lldP gene into the ldhAgene site of the cyanobacterium Synechococcus sp. PCC 7002 was constructed.

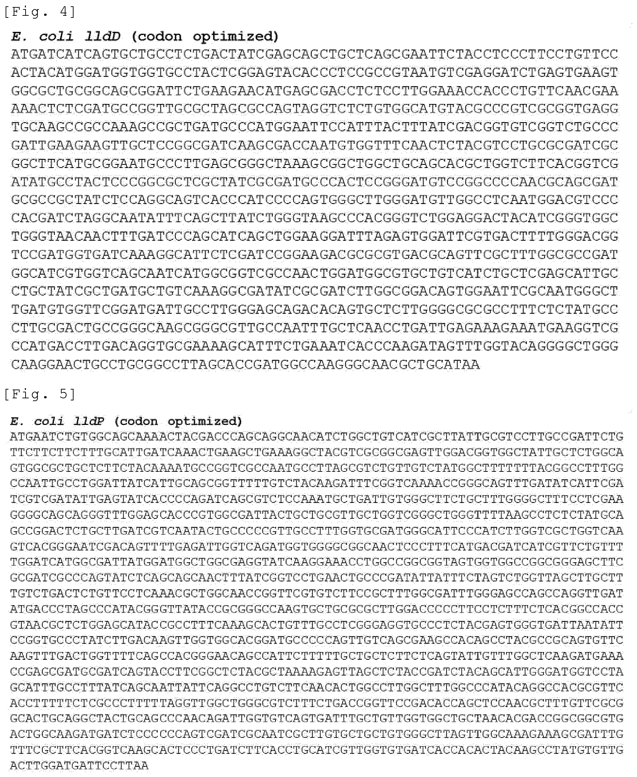

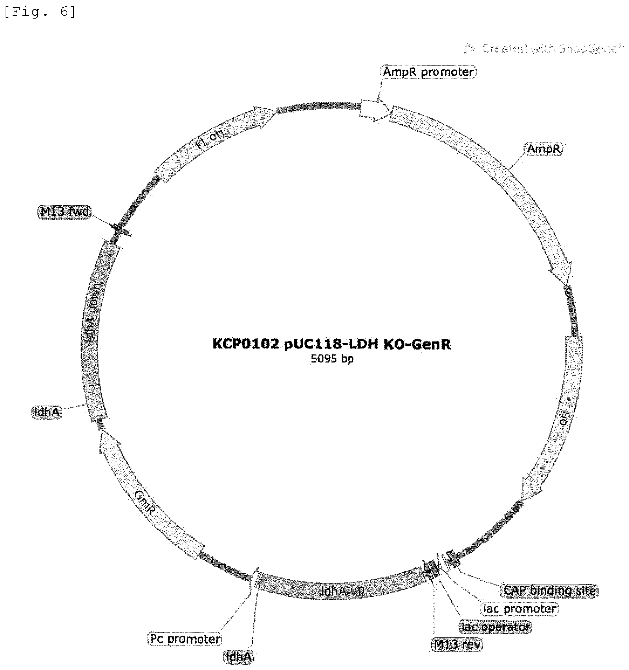

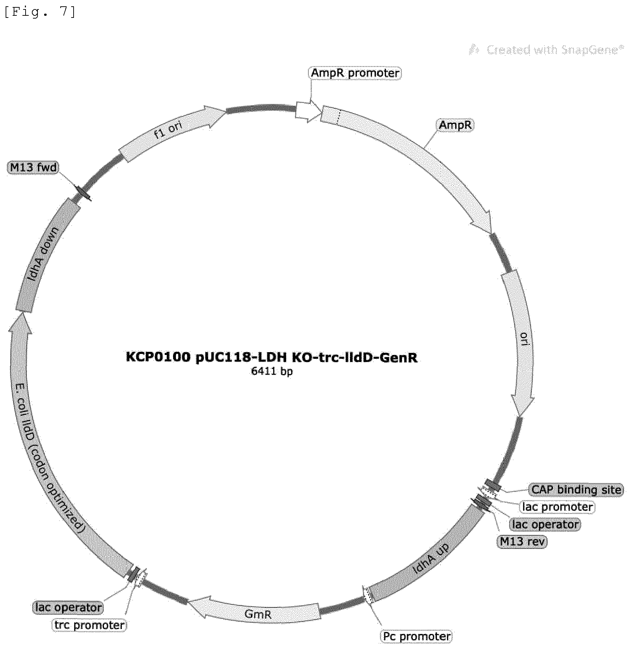

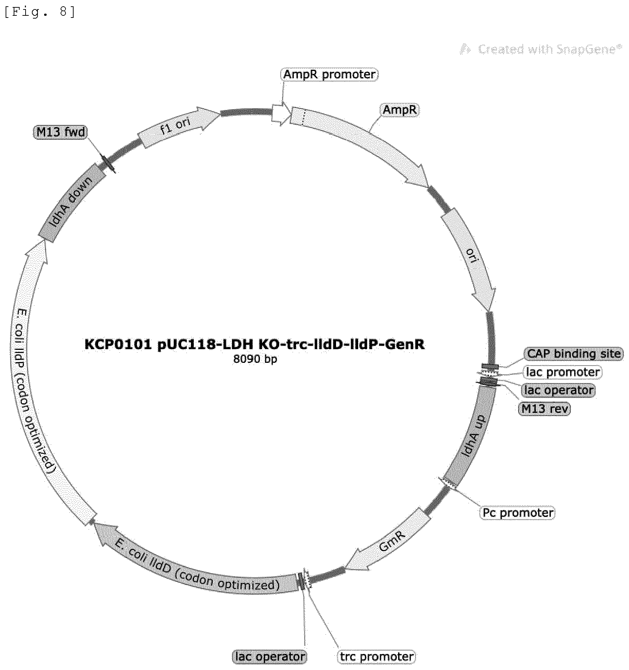

Information of LldD (NAD-independent L-lactate dehydrogenase, EC 1.1.2.3) and LldP (lactate permease) of Escherichia coli was obtained from NCBI (FIGS. 2 and 3, SEQ ID NOs: 1 and 2). Based on these amino acid sequences, a nucleic acid sequence codon-optimized using the cyanobacterium Synechococcus elongatus as a host was artificially synthesized (assigned to GenScript) (FIGS. 4 and 5, SEQ ID NOs: 3 and 4). For a vector (Plasmid name: pUC118-LDH KO-GenR, Plasmid number: KCP0102) for deleting ldhA gene (SYNPCC7002_G0164) in the cyanobacterium Synechococcus sp. PCC7002 (FIG. 6), a trc promoter and a codon-optimized lldD gene were introduced downstream of the gentamicin resistance cassette (Plasmid name: pUC118-LDH KO-trc-lldD-GenR, Plasmid number: KCP0100) (FIG. 7). Furthermore, a ribosome binding site and a codon-optimized lldP gene were introduced downstream of the lldD gene for pUC118-LDH KO-trc-lldD-GenR (Plasmid name: pUC118-LDH KO-trc-lldD-lldP-GenR, Plasmid number: KCP0101) (FIG. 8).

### (Example 2: Transformation of Blue-Green Algae)

The cyanobacterium Synechococcus sp. PCC 7002 was transformed using the plasmid constructed in Example 1, and the lldD/lldP gene was introduced into the ldhA gene site.

The cyanobacterium Synechococcus sp. PCC 7002 was cultured in MA2 medium under the conditions of 50 µmol photons/m²/s (neutral white fluorescent lamp), room temperature, and 100 rpm until the OD₇₅₀ reached about 1. One hundred µL of the culture solution was transferred to a 1.5 mL microtube, and 1 µg of the plasmid constructed in Example 1 was added. The mixture was slowly stirred at room temperature for 24 hours while being shielded from light. A filter was spread on an MA2 agar plate, and the whole amount of the cell suspension was seeded thereon. The cells were statically cultured for 2 days under conditions of 50 µmol photons/m²/s (neutral white fluorescent lamp) and 30°C. The filter was transferred to an MA2 agar plate with 40 µg/mL gentamicin. The cells were statically cultured under conditions of 50 µmol photons/m²/s (neutral white fluorescent lamp) and 30°C until colonies were formed. The cells of the colonies were cultured in MA2 medium containing 40 µg/mL gentamicin, 50 µmol photons/m²/s (neutral white fluorescent lamp) at room temperature and 100 rpm. PCR was performed using the genomic DNA of the cultured cells as a template, and it was confirmed that the lldD/lldP gene was introduced into the ldhA gene site. The plasmids and strains used are as shown in Tables 1 and 2, respectively.

**Table 1**

| |
|---|
| Plasmids used |
| Number |
| Plasmid name |
| Gene introduced into ldhA site |

**Table 2**

| |
|---|
| Strains used |
| Strain name |
| Genotype |
| Plasmid used for transformation |

### (Host)

### (Example 3: L-Lactate Usability Evaluation and Metabolic Analysis of lldD/lldP-Introduced Strain)

The lldD/lldP introduced strain prepared in Example 2 was cultured, and L-lactate usability and the like were evaluated.

The lldD/lldP introduced strain prepared in Example 2 was cultured for 3 days using a two-stage flask under conditions of MA2 medium containing 40 µg/mL of gentamicin, 100 µmol photons/m²/s (neutral white fluorescent lamp), 2% CO₂, 30°C, and 100 rpm (pre-culture). Cells were passaged from the pre-culture solution to OD₇₅₀ = 0.1, and using a two-stage flask, the cells were cultured for 14 days under conditions of MA2 medium (+20 mM L-lactate) containing 40 µg/mL of gentamicin, 100 µmol photons/m²/s (neutral white fluorescent lamp), 2% CO₂, 30°C, and 100 rpm (main culture). During the main culture period, the growth of cells was evaluated by an optical density (OD₇₅₀) at a wavelength of 750 nm, and the concentrations of nitrate, phosphate and lactate in the culture solution supernatant were measured by the following method. On day 7 of the main culture, metabolome analysis was performed by the following method.

The nitrogen source contained in the MA2 medium is only sodium nitrate. Since nitrate well absorbs light having a wavelength of 220 nm, an optical density (OD₂₂₀) at a wavelength of 220 nm was used as an index in the measurement of the concentration of nitrate in the culture solution supernatant. The cells were removed from the culture solution by centrifugation (8,000 × g, 5 min) and the OD₂₂₀ of the supernatant was measured.

For the measurement of the phosphate concentration in the culture solution supernatant, cells were removed from the culture solution by centrifugation (8,000 × g, 5 min), and measurement was performed according to a product protocol using PiBlue(trademark) Phosphate Assay Kit POPB-500 (manufactured by BioAssay Systems).

For the measurement of the lactate concentration in the culture solution supernatant, cells and cell debris were removed from the culture solution by centrifugation (8,000 × g, 5 min) and filter filtration, and the filtrate was analyzed by an HPLC apparatus (manufactured by Shimadzu Corporation) (column: Aminex HPX-87H column manufactured by Bio-Rad Laboratories, Inc., mobile phase: 5 mM sulfuric acid, temperature: 50°C, flow rate: 0.6 mL/min, analysis time: 25 min). A calibration curve was created by performing the same analysis on an L-lactate standard product having a known concentration, and the lactate concentration in the culture solution supernatant was calculated based on the calibration curve. (In this measurement method, the L-form and the D-form are not distinguished, and the same applies to the following metabolome analysis)

For the measurement of the amount of extracellular metabolites, cells were removed from the culture solution by centrifugation (8,000 × g, 5 min), and 500 µL of chloroform was added to 500 µL of the supernatant and mixed. After centrifugation (14,000 × g, 5 min, 4°C), 400 µL of the upper phase was ultrafiltered by passing through Amicon Ultra-0.5 Centrifugal Filter Unit UFC5003BK (manufactured by Merck Millipore) by centrifugation operation (14,000 × g, 90 min, 4°C). Two µL of a 100 mM PIPES/100 mM methionine sulfone mixed solution was added to 248 µL of the filtrate (used as an internal standard). This was analyzed by capillary electrophoresis-mass spectrometry (CE-MS) using G7100 CE/G6224AA LC/MSD time-of-flight systems manufactured by Agilent Technologies Inc.

For the measurement of intracellular metabolites, cells were recovered from the culture solution through a filter, immediately suspended in a methanol extract (36 µM PIPES, 36 µM methionine sulfone) precooled to -30°C, and stored at -30°C. To 500 µL of the cell suspension, 500 µL of chloroform and 200 µL of ultrapure water were added and mixed. After centrifugation (14,000 × g, 5 min, 4°C), 400 µL of the upper phase was ultrafiltered by passing through Amicon Ultra-0.5 Centrifugal Filter Unit UFC5003BK (manufactured by Merck Millipore) by centrifugation operation (14,000 × g, 90 min, 4°C). Three hundred µL of the filtrate was dried by a centrifugal evaporator and then resuspended in 20 µL ultrapure water. This was analyzed by CE-MS as well as extracellular metabolites.

### (Results)

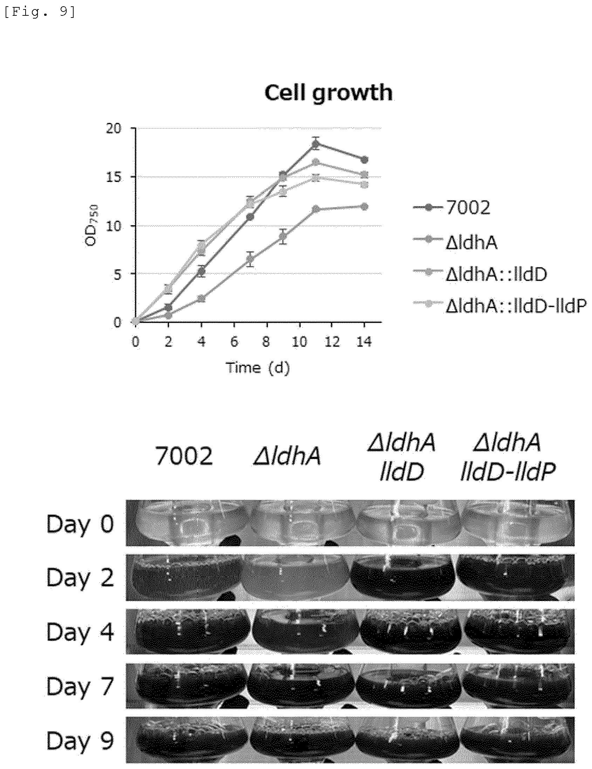

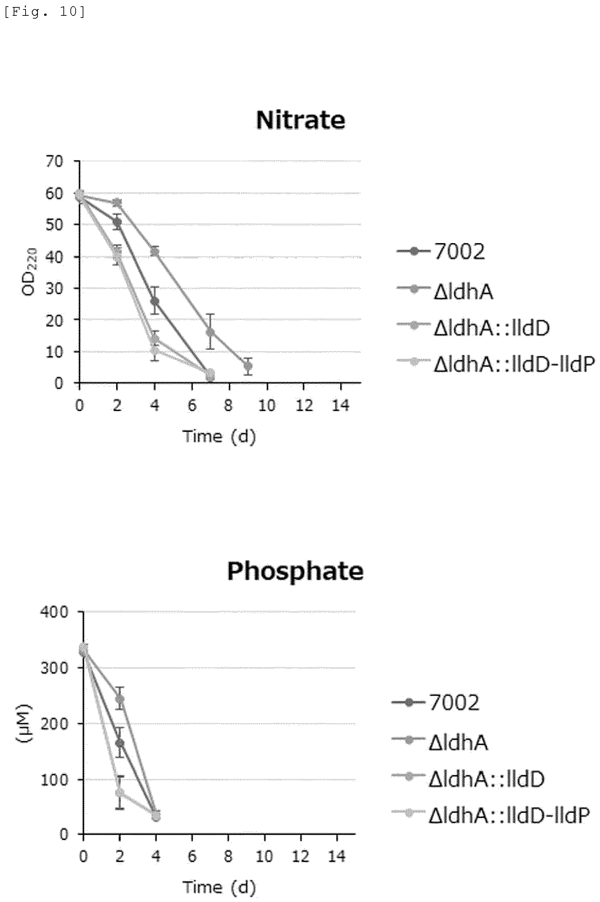

As compared with the wild-type strain PCC 7002, cell proliferation using OD₇₅₀ as an index particularly at the early stage of culture was improved in the llD/lldP introduced strain (FIG. 9). This is considered to be because the llD/lldP introduced strain utilizes not only CO₂ but also L-lactate to be used as a carbon source. In relation to the improvement in growth, the consumption of nitrate and phosphate was promoted in the llD/lldP introduced strain (FIG. 10).

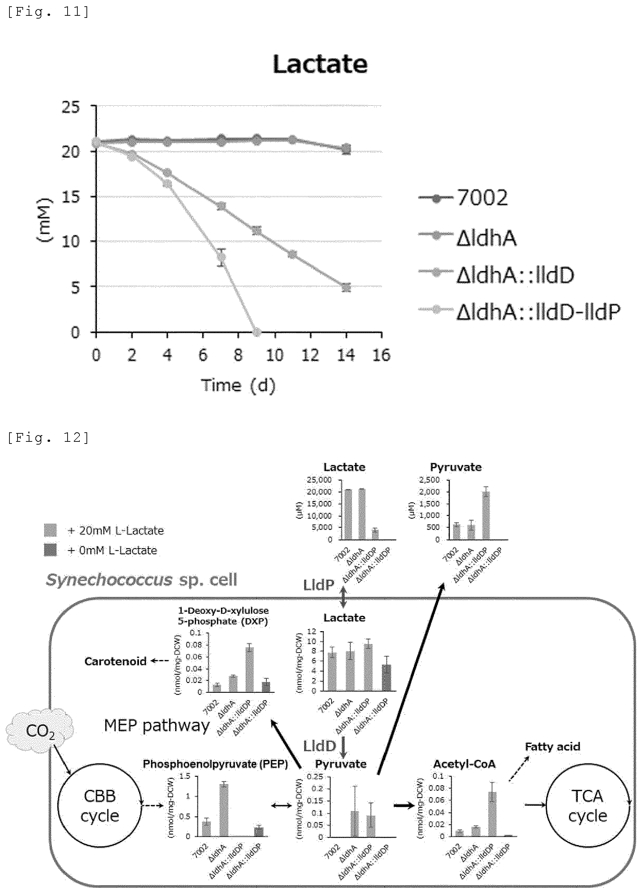

When the concentration of lactate in the culture solution supernatant was measured, the concentration of lactate in the medium was hardly changed in the PCC 7002 and ldhA-deficient strains, whereas the concentration of lactate was decreased in the lldD-introduced strain (FIG. 11). It was considered that L-lactate usability could be imparted to blue-green algae by introduction of the lldD (NAD independent L-lactate dehydrogenase, EC 1.1.2.3) gene. In the lldD/lldP introduced strain, the decrease in the lactate concentration was accelerated, and 20 mM of lactate was completely consumed on day 9 of culture. It has been found that the lldP (lactate permease) gene also functions in blue-green algae and is effective in promoting consumption of L-lactate.

On day 7 of culture, intracellular and extracellular metabolite amounts of the lldD/lldP introduced strain were analyzed (FIG. 12). As a change in intracellular metabolites related to pyruvic acid, an increase in acetyl CoA and 1-deoxy-D-xylulose-5 phosphate (DXP), which is an initial metabolite of the non-mevalonate pathway (MEP pathway), was found in L-lactate utilizing cells (lldD/lldP introduced strain + 20 mM L-lactate). This suggested that the metabolic flux derived from L-lactate was distributed to the TCA cycle and the MEP pathway. The amount of extracellular pyruvic acid was also increased in L-lactate utilizing cells, which suggested that pyruvic acid generated by LldD exits the cells.

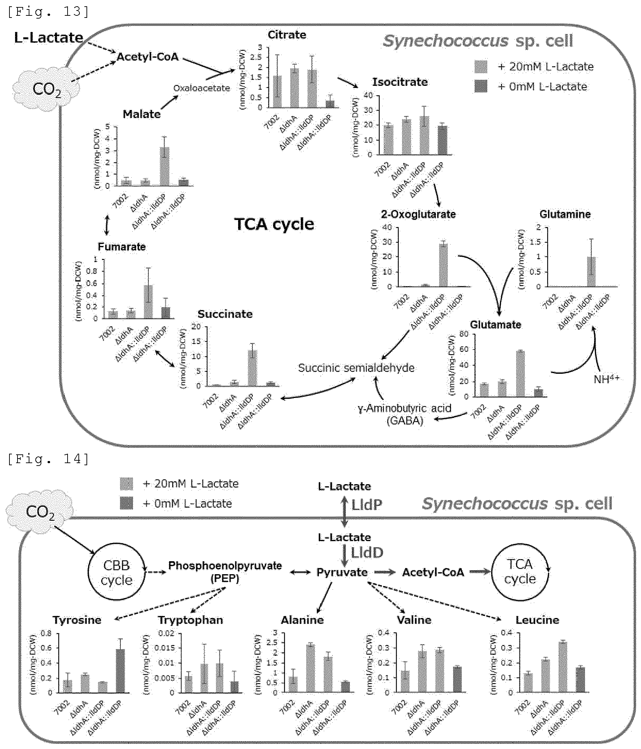

Metabolites of the TCA cycle were also analyzed (FIG. 13). In the TCA cycle, 2-oxoglutaric acid, succinic acid, fumaric acid, and malic acid were increased in L-lactate utilizing cells (lldD/lldP-introduced strain + 20 mM L-lactate). As with the increase in acetyl CoA, it is suggested that the metabolic flux derived from L-lactate is distributed to the TCA cycle. In addition, in association with the TCA cycle, an increase in glutamine and glutamic acid was also observed in the L-lactate utilizing cells. It is suggested that the present technique is useful in the production of these amino acids. Since a large amount of 2-oxoglutaric acid which is a precursor of glutamine and glutamic acid is accumulated in cells, enhancement of glutamine/glutamic acid production can be expected by enhancing the activity of glutamine synthetase (GS) or glutamic acid synthetase (GOGAT) by a genetic engineering technique.

In amino acids other than glutamine and glutamic acid, the intracellular amounts of alanine, valine, and leucine derived from pyruvic acid were increased (FIG. 14). These amino acids can also be expected to be produced using the present technique.

### (Example 4: Example of Novel Waste-Free Food Production)

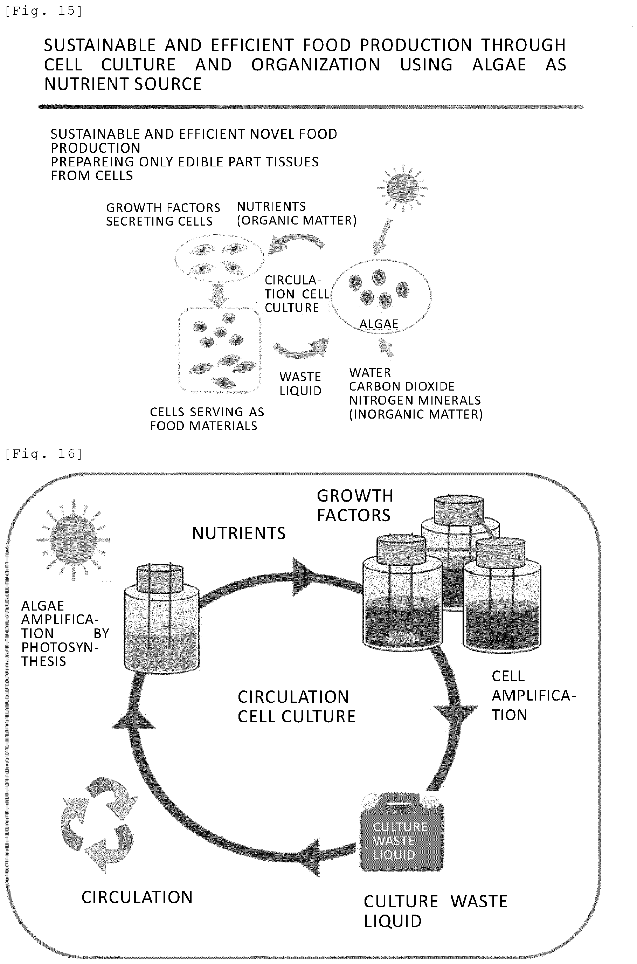

In the present example, an application example of the technique of the present disclosure for new waste-free food production is illustrated using FIG. 15.

In the present example, only the edible part tissue is produced.

In the present example, algae, growth factor secreting cells, and muscle cells, which are examples of the present disclosure, are used as an example to constitute a system (circulating system).

First, a component (organic matter) derived from algae is used as a nutrient source in growth factor secreting cells. The growth factor excreted from the growth factor secreting cells acts on muscle cells that can use the growth factor contained in this system, and the muscle cells proliferate. Using the culture waste liquid of muscle cells, a component (for example, L-lactate and the like) contained in the waste liquid is used again in algae, and carbon dioxide, nitrogen, minerals, and the like are taken in appropriately, such that circular cell culture (CCC) is completed (FIG. 15).

The muscle cells produced in this way can be collected and appropriately molded to be used as food.

### (Example 5: Another Example of Demonstration of CCC)

In the present example, similarly to Example 4, a demonstration of concept of circular cell culture (CCC) is performed using another system as illustrated in FIG. 16.

Here, a demonstration example is carried out by CCC with the target of an algal decomposition rate of 50%, proliferation of chick embryo-derived muscle cells (doubling time < 96 h, 1.2 times/day), and a culture waste liquid circulating rate of 30%.

In the present example, a system that goes through nutrient supply, cell growth, waste circulating is prepared. Here, a system using algae (for example, blue-green algae), chicken muscle cells, and growth factor secreting cells of the present disclosure is assumed (FIG. 16).

Algae cells take up carbon dioxide, nitrogen, minerals and provide nutrient sources such as glucose, amino acids, and vitamins. Here, for nutrient source supply, ultrasonic treatment, catalytic chemistry, and genetic engineering techniques can be combined to extract the nutrient source required for chicken muscle cells from algae. The chick muscle cells can be grown under appropriate conditions. Ammonium, L-lactate, and residual proteins excreted along with the cell growth are subjected to waste liquid circulating, and some of them can be used for the growth of blue-green algae. As a result, it is possible to show the feasibility of mass production in view of energy efficiency, production efficiency, and production cost.

### (Example 6: Production Example of Cultured Meat)

An example of the cultured meat is shown in the present example.

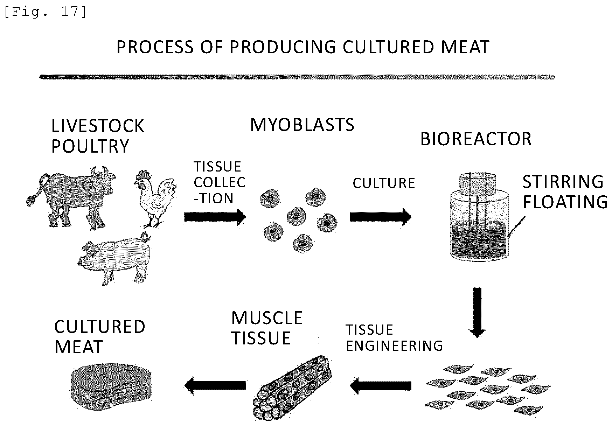

In the present example, as shown in FIG. 17, a large amount of culture solution and waste liquid associated with an increase in culture food production are circulated and used.

### (Materials)

In the present example, the system in Examples 4 and 5 is used, but bovine muscle cells are used as muscle cells. As the growth factor, a commercially available growth factor may be used, or the growth factor may be produced by cells as described in Examples 4 and 5. This is because a sufficient environmental load reduction effect is expected only by the treatment of the waste liquid.

### Configuration of culture solution:

### Basic nutrients (sugar, amino acid, and vitamin)

In the case of grain origin, there is a risk due to an increase in environmental load and environmental change, and thus this is replaced with a nutrient source derived from blue-green algae.

### Cell growth factors:

### Those derived from livestock (such as cattle) serum may be used, or growth factor producing cells may be used in combination.

Here, the culture waste liquid includes ammonium, L-lactate, phosphate, and the like, and conventionally, the environmental load is applied as sewage, but is eliminated in the present disclosure.

### (Example 7: Production Example of Plastic)

The present example demonstrates an example of a plastic (such as PLA).

For example, it is possible to produce a high performance biomass plastic from polysaccharides using the cell system of the present disclosure. Examples of the polysaccharides include, but are not limited to, natural polysaccharides, starch, cellulose, glucomannan, paramylon produced by Euglena, and non-natural polysaccharides.

When glucose (monosaccharide) is formed from the polysaccharides, lactate is synthesized from glucose by Lactobacillus or the like, and then polylactate is formed by an appropriate synthesis reaction. Alternatively, microbially produced polyesters can be produced from glucose.

From polysaccharides, polysaccharide plastics can be produced by a modification such as a chemical modification.

### (Example 8: Selection of Animal Cells and Blue-Green Algae Cells)

In the present example, an example in which circulation cell culture is performed using various cells will be described.

In the present example, the system in Examples 4 and 5 is applied, but any one or more of the following are used as organisms. The organism may be an unmodified organism. Crocosphaera watsonii is useful for use in CCC because it has been reported that it can accumulate starch or immobilize atmospheric nitrogen.

### [Unmodified Organism Possibly Having NAD-Independent L-Lactate Dehydrogenase]

- (Blue-Green Algae) Crocosphaera watsonii, Trichodesmium erythraeum, Rivularia sp.
- (Microalgae) Aureococcus anophagefferens

### [Organism in Which NAD-Independent L-Lactate Dehydrogenase or L-Lactate Permease Is Introduced and Utilized]

- (Blue-Green Algae) Anabaena sp., Synechocystis sp., Synechococcus elongatus, and the like
- (Microalgae) Chlorococcum littorale, Chlorella sorokiniana, Chlorella vulgaris, Chlamydomonas sp., Cyanidioschyzon merolae, and the like

Using these cells, circulation cell culture is performed in the same system as in Examples 4 and 5.

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present specification are to be incorporated by reference in the present specification in the same manner as the contents is specifically described in the present specification. The present application claims priority to Japanese Patent Application No. 2021-198872 filed on December 7, 2021 to the Japanese Patent Office, the entire contents of which are incorporated herein by reference in the same manner as if the entire contents constituted the present application.

### Industrial Applicability

According to the present disclosure, in meat production by cell culture, which is expected to be realized, L-lactate, which is a main a component excreted from cultured animal cells, is consumed by microalgae and blue-green algae, and a component derived from the microalgae and blue-green algae are used as a nutrient source of the cultured animal cells, such that it is possible to construct a circulation system that minimizes waste and environmental load. As a result, it is possible to develop a circulation cell culture system having high efficiency and low environmental load, and thus application is expected in industrial fields such as cell agriculture, culture food production, and circulation systems. Sequence Listing Free Text

SEQ ID NO: 1: Amino acid sequence of LldD in Escherichia coli
SEQ ID NO: 2: Amino acid sequence of LldP in Escherichia coli
SEQ ID NO: 3: Base sequence of lldD codon-optimized using Synechococcus elongatus as host
SEQ ID NO: 4: Base sequence of lldP codon-optimized using Synechococcus elongatus as host

## Claims

1. An organism or a cultured cell which undergoes a modification, wherein the modification includes imparting or enhancing L-lactate utilization ability in the organism or the cultured cell.

2. The organism or the cultured cell according to claim 1, wherein the organism or the cultured cell includes blue-green algae, microalgae, a yeast, E. coli, Bacillus subtilis, corynebacterium, actinomycetes, filamentous fungus, Bacillus subtilis, and Lactobacillus.

3. The organism or the cultured cell according to claim 1 or 2, wherein the modification includes introduction of a gene that utilizes L-lactate.

4. The organism or the cultured cell according to any one of claims 1 to 3, wherein the modification includes introduction of a gene that encodes NAD-independent L-lactate dehydrogenase (EC 1.1.2.3).

5. The organism or the cultured cell according to any one of claims 1 to 4, wherein the modification includes introduction of lldD gene derived from E. coli.

6. The organism or the cultured cell according to any one of claims 1 to 5, wherein the modification includes introduction of a gene that imparts or enhances membrane permeability of L-lactate.

7. The organism or the cultured cell according to any one of claims 1 to 6, wherein the modification includes introduction of a gene that encodes lactate permease.

8. The organism or the cultured cell according to any one of claims 1 to 7, wherein the modification includes introduction of lldP gene derived from E. coli.

9. The organism or the cultured cell according to any one of claims 1 to 8, wherein the modification includes attenuation or deletion of a gene that synthesizes L-lactate or D-lactate.

10. The organism or the cultured cell according to any one of claims 1 to 9, wherein the modification includes attenuation or deletion of a gene that encodes D-lactate dehydrogenase.

11. The organism or the cultured cell according to any one of claims 1 to 10, wherein the modification includes attenuation or deletion of ldhA gene.

12. The organism or the cultured cell according to any one of claims 1 to 11, wherein the organism or the cultured cell has a pyruvic acid metabolism system and/or a TCA cycle.

13. The organism or the cultured cell according to any one of claims 1 to 12, wherein the organism or the cultured cell has a synthetic pathway of succinic acid, 2-oxoglutaric acid, 3-hydroxypropionic acid, acrylic acid, and/or acetic acid.

14. The organism or the cultured cell according to any one of claims 1 to 13, wherein the organism or the cultured cell has an amino acid synthetase and/or an amino acid synthetic system, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane .

15. The organism or the cultured cell according to claim 14, wherein the amino acid includes glutamine, glutamic acid, alanine, valine, and leucine.

16. A method for culturing at least two types of cells including a cell X and a cell Y in a circulation manner, the method comprising:
a step (a) of providing a component excreted from the cell X to the cell Y;
a step (b) of providing a component derived from the cell Y to the cell X;
a step (c) of culturing the cell X and the cell Y; and
a step (d) of repeating the steps (a) to (c) as necessary, wherein
at least one of the components excreted from the cell X is an assimilable a component of the cell Y, and
at least one of the components derived from the cell Y is a nutritional a component of the cell X.

17. A method for producing a desired product, the method comprising a step of performing the method according to claim 16, wherein
at least one of the cell X or Y produces the desired product.

18. The method according to claim 16 or 17, wherein the cells X and Y include animal cells and blue-green algae cells.

19. The method according to any one of claims 16 to 18, wherein the component excreted from the cell X and the component derived from the cell Y include L-lactate and glutamine or glutamate, respectively.

20. The method according to any one of claims 16 to 19, wherein the culturing step includes culturing the cell X and the cell Y in different places.

21. A method for culturing animal cells by circulating component excreted from an animal cell culture by an organism, the method comprising:
a step (a) of consuming the excreted a component, the organism having utilization ability of the component;
a step (b) of harvesting a nutrient source of the animal cells from a culture solution of the organism;
a step (c) of culturing the animal cells using the nutrient source; and
a step (d) of repeating the steps (a) to (c) as necessary.

22. The method according to claim 21, wherein the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B1, vitamin B2, vitamin B3, vitamin B5, vitamin B6, folate, choline, starch, glycogen, and cellulose.

23. The method according to claim 21 or 22, wherein the organism has an ability to utilize a component to be excreted to produce the nutrient source, or the organism is modified to impart or enhance utilization ability of the component.

24. The method according to any one of claims 21 to 23, wherein a component to be excreted include L-lactate, carbon dioxide, ammonium, urea, or phosphate.

25. The method according to any one of claims 21 to 24, wherein a component to be excreted is L-lactate, ammonium, or urea, and is toxic to the animal cells.

26. The method according to any one of claims 21 to 25, wherein the organism has a pyruvic acid metabolism system and/or a TCA cycle.

27. The method according to any one of claims 21 to 26, wherein the organism has a synthetic pathway of succinic acid, 2-oxoglutaric acid, 3-hydroxypropionic acid, acrylic acid, and/or acetic acid.

28. A method for producing cultured meat by circulating a component excreted from an animal cell culture by an organism to culture animal cells, the method comprising:
a step (a) of causing an organism to consume a component to be excreted, the organism having utilization ability of the component;
a step (b) of recovering a nutrient source of the animal cells from the organism;
a step (c) of culturing the animal cells using the nutrient source;
a step (d) of repeating the steps (a) to (c) as necessary; and
a step (e) of subjecting the cultured animal cells to three-dimensional organization and molding the three-dimensionally organized animal cells into cultured meat.

29. The method according to claim 28, wherein the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B1, vitamin B2, vitamin B3, vitamin B5, vitamin B6, folate, choline, starch, glycogen, and cellulose.

30. The method according to claim 28 or 29, wherein the organism has an ability to utilize a component to be excreted to produce the nutrient source, or the organism is modified to impart or enhance utilization ability of the component.

31. The method according to any one of claims 28 to 30, wherein a component to be excreted includes L-lactate, carbon dioxide, ammonium, urea, or phosphate.

32. The method according to any one of claims 28 to 31, wherein a component to be excreted is L-lactate, ammonium, or urea, and is toxic to the animal cells.

33. The method according to any one of claims 28 to 32, wherein the organism has a pyruvic acid metabolism system and/or a TCA cycle.

34. The method according to any one of claims 28 to 33, wherein the organism has a synthetic pathway of succinic acid, 2-oxoglutaric acid, 3-hydroxypropionic acid, acrylic acid, and/or acetic acid.

35. A method for producing an amino acid and/or a desired product in an organism or a cultured cell using L-lactate excreted from an animal cell culture, the method comprising:
a step of providing the L-lactate to the organism or the cultured cell, the organism or the cultured cell having L-lactate utilization ability or being modified to impart or enhance L-lactate utilization ability in the organism or the cultured cell;
a step of culturing the organism or the cultured cell under conditions in which L-lactate is utilized; and
a step of recovering the amino acid as necessary.

36. The method according to claim 35, wherein the organism or the cultured cell includes blue-green algae, microalgae, a yeast, E. coli, Bacillus subtilis, corynebacterium, actinomycetes, filamentous fungus, Bacillus subtilis, and Lactobacillus.

37. The method according to claim 35 or 36, wherein the modification includes introduction of a gene that utilizes L-lactate.

38. The method according to any one of claims 35 to 37, wherein the modification includes introduction of a gene that encodes NAD-independent L-lactate dehydrogenase (EC 1.1.2.3).

39. The method according to any one of claims 35 to 38, wherein the modification includes introduction of lldD gene derived from E. coli.

40. The method according to any one of claims 35 to 39, wherein the modification includes introduction of a gene that imparts or enhances membrane permeability of L-lactate.

41. The method according to any one of claims 35 to 40, wherein the modification includes introduction of a gene that encodes lactate permease.

42. The method according to any one of claims 35 to 41, wherein the modification includes introduction of lldP gene derived from E. coli.

43. The method according to any one of claims 35 to 42, wherein the modification includes attenuation or deletion of a gene that synthesizes L-lactate or D-lactate.

44. The method according to any one of claims 35 to 43, wherein the modification includes attenuation or deletion of a gene that encodes D-lactate dehydrogenase.

45. The method according to any one of claims 35 to 44, wherein the modification includes attenuation or deletion of ldhA gene.

46. The method according to any one of claims 35 to 45, wherein the organism or the cultured cell has a pyruvic acid metabolism system and/or a TCA cycle.

47. The method according to any one of claims 35 to 46, wherein the organism or the cultured cell has a synthetic pathway of succinic acid, 2-oxoglutaric acid, 3-hydroxypropionic acid, acrylic acid, and/or acetic acid.

48. The method according to any one of claims 35 to 47, wherein the organism or the cultured cell has an amino acid synthetase and/or an amino acid synthetic system, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane.

49. The method according to claim 48, wherein the amino acid includes glutamine, glutamic acid, alanine, valine, and leucine.

50. The method according to any one of claims 35 to 49, wherein the desired product includes D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, and butane.

51. A method for producing a processed product of an amino acid and/or a desired product produced by an organism or a cultured cell using L-lactate excreted from an animal cell culture, the method comprising:
a step of providing the L-lactate to the organism or the cultured cell, the organism or the cultured cell having L-lactate utilization ability or being modified to impart or enhance L-lactate utilization ability in the organism or the cultured cell;
a step of culturing the organism or the cultured cell under conditions in which L-lactate is utilized;
a step of recovering the amino acid and/or the desired product; and
a step of processing the amino acid and/or the desired product.

52. The method according to claim 51, wherein the organism or the cultured cell includes blue-green algae, microalgae, a yeast, E. coli, Bacillus subtilis, corynebacterium, actinomycetes, filamentous fungus, Bacillus subtilis, and Lactobacillus.

53. The method according to claim 51 or 52, wherein the modification includes introduction of a gene that utilizes L-lactate.

54. The method according to any one of claims 51 to 53, wherein the modification includes introduction of a gene that encodes NAD-independent L-lactate dehydrogenase (EC 1.1.2.3).

55. The method according to any one of claims 51 to 54, wherein the modification includes introduction of lldD gene derived from E. coli.

56. The method according to any one of claims 51 to 55, wherein the modification includes introduction of a gene that imparts or enhances membrane permeability of L-lactate.

57. The method according to any one of claims 51 to 56, wherein the modification includes introduction of a gene that encodes lactate permease.

58. The method according to any one of claims 51 to 57, wherein the modification includes introduction of lldP gene derived from E. coli.

59. The method according to any one of claims 51 to 58, wherein the modification includes attenuation or deletion of a gene that synthesizes L-lactate or D-lactate.

60. The method according to any one of claims 51 to 59, wherein the modification includes attenuation or deletion of a gene that encodes D-lactate dehydrogenase.

61. The method according to any one of claims 51 to 60, wherein the modification includes attenuation or deletion of ldhA gene.

62. The method according to any one of claims 51 to 61, wherein the organism or the cultured cell has a pyruvic acid metabolism system and/or a TCA cycle.

63. The method according to any one of claims 51 to 62, wherein the organism or the cultured cell has a synthetic pathway of succinic acid, 2-oxoglutaric acid, 3-hydroxypropionic acid, acrylic acid, and/or acetic acid.

64. The method according to any one of claims 51 to 63, wherein the organism or the cultured cell has an amino acid synthetase and/or an amino acid synthetic system, and/or a synthetase and/or a synthetic system for D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, or butane.

65. The method according to claim 64, wherein the amino acid includes glutamine, glutamic acid, alanine, valine, and leucine.

66. The method according to any one of claims 51 to 65, wherein the desired product includes D-lactic acid, 3-hydroxypropionic acid, acrylic acid, formic acid, acetic acid, ethanol, 2,3-butanediol, butanol, acetone, isobutyric acid, isobutanol, isobutylene, butyric acid, 1-butanol, propane, caproic acid, pentane, citric acid, succinic acid, fumaric acid, malic acid, itaconic acid, γ-aminobutyric acid, γ-butyrolactam, ornithine, putrescine, 6-aminocaproic acid, ε-caprolactam, ethylene, γ-butyrolactone, 1,4-butane diol, levulinic acid, adipic acid, cadaverine, 5-aminovaleric acid, δ-valerolactam, 1-propanol, and butane.

67. A method for breeding an organism or a cultured cell using a component excreted from an animal cell culture, the method comprising:
a step (A) of providing a component excreted from animal cells to a candidate organism or cultured cell;
a step (B) of determining whether the organism or the cultured cell consumes the excreted a component or enhances the consumption;
a step (C) of selecting an organism or a cultured cell in which consumption is imparted or enhanced among the organisms or cultured cells, wherein
the selected organism or cultured cell is determined to have utilization ability of a component or to be modified to impart or enhance utilization ability of a component in the organism or the cultured cell.

68. The method according to claim 67, further comprising producing a nutrient source through utilization.

69. The method according to claim 68, wherein the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B1, vitamin B2, vitamin B3, vitamin B5, vitamin B6, folate, choline, starch, glycogen, and cellulose.

70. The method according to any one of claims 67 to 69, wherein a component to be excreted includes L-lactate, carbon dioxide, ammonium, urea, or phosphate.

71. The method according to any one of claims 67 to 70, wherein a component to be excreted is L-lactate, ammonium, or urea, and is toxic to the animal cells.

72. The method according to any one of claims 67 to 71, wherein the organism has a pyruvic acid metabolism system and/or a TCA cycle.

73. The method according to any one of claims 67 to 72, wherein the organism has a synthetic pathway of succinic acid, 2-oxoglutaric acid, 3-hydroxypropionic acid, acrylic acid, and/or acetic acid.

74. A method for maintaining an organism or a cultured cell using a component excreted from an animal cell culture, the method comprising:
a step (a) of causing the organism or the cultured cell to consume a component to be excreted, the organism or the cultured cell having utilization ability of a component or being modified to impart or enhance utilization ability of a component in the organism or the cultured cell;
a step (b) of recovering a nutrient source of the animal cells from the organism or the cultured cell as necessary;
a step (c) of culturing the animal cells using the nutrient source as necessary; and
a step (d) of providing a component excreted from the animal cells to the organism or the cultured cell.

75. The method according to claim 74, wherein the nutrient source includes glucose, glutamic acid, glutamine, alanine, arginine, asparagine, aspartic acid, cysteine, glycine, histidine, isoleucine, leucine, lysine, methionine, phenylalanine, proline, serine, threonine, tryptophan, tyrosine, valine, pyruvic acid, vitamin B1, vitamin B2, vitamin B3, vitamin B5, vitamin B6, folate, choline, starch, glycogen, and cellulose.

76. The method according to claim 74 or 75, wherein a component to be excreted includes L-lactate, carbon dioxide, ammonium, urea, or phosphate.

77. The method according to any one of claims 74 to 76, wherein a component to be excreted is L-lactate, ammonium, or urea, and is toxic to the animal cells.

78. The method according to any one of claims 74 to 77, wherein the organism has a pyruvic acid metabolism system and/or a TCA cycle.

79. The method according to any one of claims 74 to 78, wherein the organism has a synthetic pathway of succinic acid, 2-oxoglutaric acid, 3-hydroxypropionic acid, acrylic acid, and/or acetic acid.

80. A gene used for breeding the organism or the cultured cell which undergoes a modification according to any one of claims 1 to 15 or a kit comprising the gene.

81. A method for evaluating a modified strain, the method comprising:
a step of preparing the organism or the cultured cell according to any one of claims 1 to 15; and
a step of confirming the presence of the modification in the organism or the cultured cell.

82. The method according to claim 81, wherein the step of confirming the presence of the modification includes supplying carbon dioxide used in photosynthesis to the organism or the cultured cell at a constant concentration.

83. A method for producing a plastic raw material from an organism or a cultured cell using L-lactate excreted from an animal cell culture, the method comprising:
a step of providing the L-lactate to the organism or the cultured cell, the organism or the cultured cell having L-lactate utilization ability or being modified to impart or enhance L-lactate utilization ability in the organism or the cultured cell;
a step of culturing the organism or the cultured cell under conditions in which L-lactate is utilized; and
a step of recovering a component used as a plastic raw material from the organism or the cultured cell, wherein
the organism or the cultured cell is capable of synthesizing a component used as a plastic raw material, or a gene required for synthesis of a component used as a plastic raw material is introduced into the organism or the cultured cell.

84. A method for producing a lactate derivative or a derivative thereof, the method comprising:
a step of providing L-lactate to an organism which undergoes a modification, the modification including imparting or enhancing L-lactate utilization ability in the organism;
a step of culturing the organism under conditions in which L-lactate is utilized; and
a step of recovering a desired lactate derivative or a derivative thereof as necessary.

85. A method for producing pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid, the method comprising:
a step of providing L-lactate to an organism which undergoes a modification, the modification including imparting or enhancing L-lactate utilization ability in the organism;
a step of culturing the organism under conditions in which L-lactate is utilized; and
a step of recovering a desired pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid as necessary.

86. The method according to claim 85, further comprising a step of recovering a substance released extracellularly.

87. A method for producing a processed product of pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid, the method comprising:
a step of providing L-lactate to an organism which undergoes a modification, the modification including imparting or enhancing L-lactate utilization ability in the organism;
a step of culturing the organism under conditions in which L-lactate is utilized;
a step of recovering a desired pyruvic acid, acetyl CoA, phosphoenolpyruvate, succinic acid, 2-oxoglutaric acid, hydroxypropionic acid, acrylic acid, or acetic acid, or a derivative of pyruvic acid, acetyl CoA, phosphoenolpyruvate, succinic acid, 2-oxoglutaric acid, hydroxypropionic acid, acrylic acid, or acetic acid as necessary; and
a step of processing the pyruvic acid, acetyl CoA, phosphoenolpyruvate, succinic acid, 2-oxoglutaric acid, hydroxypropionic acid, acrylic acid, or acetic acid, or the derivative of pyruvic acid, acetyl CoA, phosphoenolpyruvate, succinic acid, 2-oxoglutaric acid, hydroxypropionic acid, acrylic acid, or acetic acid.
